(19) Europäisches Patentamt
European Patent Office
Office européen des brevets

(11) EP 0 877 607 B1

(12) # FASCICULE DE BREVET EUROPEEN

(45) Date de publication et mention de la délivrance du brevet:
**15.11.2006 Bulletin 2006/46**

(21) Numéro de dépôt: **97902407.2**

(22) Date de dépôt: **30.01.1997**

(51) Int Cl.:
*A61K 31/055* (2006.01) *A61K 31/11* (2006.01)
*A61K 31/085* (2006.01) *A61K 31/09* (2006.01)
*A61K 31/05* (2006.01) *A61K 31/135* (2006.01)
*A61K 31/10* (2006.01) *A61K 31/40* (2006.01)
*C07C 39/367* (2006.01) *C07C 47/57* (2006.01)
*C07C 43/23* (2006.01)

(86) Numéro de dépôt international:
**PCT/FR1997/000183**

(87) Numéro de publication internationale:
**WO 1997/027846 (07.08.1997 Gazette 1997/34)**

(54) **COMPOSES BIPHENYLES ET LEUR UTILISATION COMME AGENTS ESTROGENIQUES**
BIPHENYLVERBINDUNGEN SOWIE IHRE ANWENDUNG ALS ÖSTROGENE MITTEL
BIPHENYL COMPOUNDS AND USE THEREOF AS OESTROGENIC AGENTS

(84) Etats contractants désignés:
**AT BE CH DE DK ES FI FR GB GR IE IT LI LU NL PT SE**

(30) Priorité: **01.02.1996 FR 9601211**

(43) Date de publication de la demande:
**18.11.1998 Bulletin 1998/47**

(73) Titulaire: **Aventis Pharma S.A.**
**92160 Antony (FR)**

(72) Inventeurs:
- **LESUISSE, Dominique**
**F-75018 Paris (FR)**
- **TEUTSCH, Jean-Georges**
**F-93500 Pantin (FR)**

(74) Mandataire: **Hirsch & Associés**
**58, avenue Marceau**
**75008 Paris (FR)**

(56) Documents cités:
**FR-A- 2 189 036**

- **PATENT ABSTRACTS OF JAPAN vol. 017, no. 327 (C-1073), 22 Juin 1993 & JP 05 032579 A (BANYU PHARMACEUTICAL), 9 Février 1993,**
- **CHEMISCHE BERICHTE, vol. 111, no. 4, 1978, WEINHEIM, DE, pages 1323-1329, XP002016695 G. HÄFELINGER, ET AL.: "Untersuchungen zur Anwendbarkeit der Mesitylgruppe als NMR-Sonde zum Nachweis von Ringstrom-Anisotropieeffekten, II. Substituenteneffekt bei p-substituierten Mesitylbenzolderivaten"**
- **JOURNAL OF THE CHEMICAL SOCIETY, no. 7, Juillet 1961, LETCHWORTH, GB, pages 2890-2902, XP002016696 A. DIBBO, ET AL.: "The synthesis of thyroxine and related compounds. Part XVII. The preparation of some additional compounds related to thyroxine"**
- **RECUEIL DES TRAVAUX CHIMIQUES DES PAYS-BAS, vol. 72, no. 8, Août 1953, AMSTERDAM, NL, pages 774-780, XP002016690 N.P. BUU-HOI, ET AL.: "Analogues de la p-hydroxypropiophénone dans la série du biphényle"**
- **JOURNAL OF MEDICINAL CHEMISTRY, vol. 16, no. 5, Mai 1973, WASHINGTON, DC, US, pages 490-493, XP002016691 J.S. KALTENBRONN: "4- and 5-Aryl-1-naphthaleneacetic acids as antiinflammatory agents"**
- **HELVETICA CHIMICA ACTA, vol. 58, no. 1, 29 Janvier 1975, BASEL, CH, pages 268-278, XP002016692 A. FRANKE, ET AL.: "p-Substiuierte beta-Methyl-Zimtsäurederivate"**
- **BULLETIN OF THE CHEMICAL SOCIETY OF JAPAN, vol. 30, no. 9, Décembre 1957, TOKYO, JP, pages 958-961, XP002016698 T. SATO, ET AL.: "Oestrogenic biphenyls. IV. 3'-Alkyl-4-methoxybiphenyl-4'- carboxylic acids"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 877 607 B1

## Description

La présente invention concerne l'application à titre de médicaments de composés biphényles, les compositions pharmaceutiques les contenant, des composés biphényles nouveaux, leur procédé de préparation et les intermédiaires de ce procédé.

L'invention a pour objet à titre de médicaments les composés de formule générale (I) :

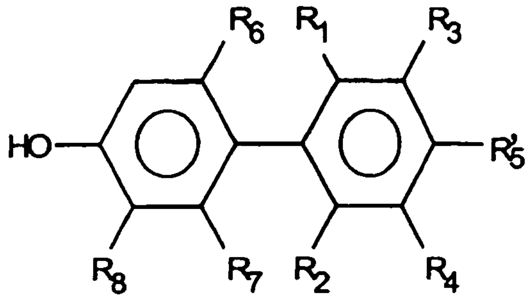

(I)

dans laquelle $R_1$ et $R_2$, identiques ou différents, représentent un atome d'hydrogène, un atome d'halogène, un radical hydroxyle, un radical trifluorométhyle, un radical nitro, un radical amino, un radical alkyloxy, alkylthio, alkylamino ou dialkylamino, dans lesquels alkyle renferme de 1 à 8 atomes de carbone, un groupement $-NR_AR_B$, dans lequel $R_A$ et $R_B$ forment avec l'atome d'azote auquel ils sont liés un hétérocycle saturé ou insaturé de 5 à 6 chaînons comportant éventuellement un autre hétéroatome choisi parmi N, O et S, un radical alkyle, alkényle ou alkynyle, linéaire ou ramifié, renfermant chacun au plus 8 atomes de carbone et éventuellement substitué, un radical aryle renfermant de 6 à 14 atomes de carbone et éventuellement substitué, un radical aralkyle renfermant de 7 à 15 atomes de carbone et éventuellement substitué, ou un radical CH(OH)-Y ou C(O)-Y dans lequel Y représente un radical alkyle, alkényle ou alkynyle renfermant de 1 à 8 atomes de carbone, substitué ou non substitué ou un groupement aryle renfermant de 6 à 14 atomes de carbone, substitué ou non substitué ou bien $R_1$ peut former avec $R_3$ un groupement- CH=CH-CH=CH-, $R_3$ et $R_4$, identiques ou différents, représentent un atome d'hydrogène, un atome d'halogène ou un radical alkyle renfermant de 1 à 8 atomes de carbone, ou $R_3$ peut former avec $R_1$ un groupement -CH=CH-CH=CH-, $R_6$ et $R_7$ identiques ou différents, représentent un atome d'hydrogène ou un atome d'halogène, $R_8$ représente un atome d'hydrogène ou un radical benzyle éventuellement substitué et $R'_5$ représente un radical $CH_2OH$, ainsi que les sels d'addition avec les acides et les bases pharmaceutiquement acceptables,

Par atome halogène on entend le fluor, l'iode, le brome et le chlore.

Par radical alkyloxy renfermant de 1 à 8 atomes de carbone, on entend de préférence le radical choisi parmi méthoxy, éthoxy, propoxy, butoxy et pentoxy.

Par radical alkylthio renfermant de 1 à 8 atomes de carbone, on entend de préférence le radical choisi parmi méthylthio, éthylthio, propylthio, isopropylthio et butylthio.

Par radical alkylamino renfermant de 1 à 8 atomes de carbone on entend de préférence le radical choisi parmi méthylamino, éthylamino, propylamino, butylamino, pentylamino.

Par radical dialkylamino renfermant chacun de 1 à 8 atomes de carbone on entend de préférence le radical choisi parmi diméthylamino, diéthylamino et méthyléthylamino.

Par groupement $-NR_AR_B$ on entend de préférence le groupement choisi parmi pyrrolidinyle, pipéridinyle, pipérazinyle, morpholinyle, thiamorpholinyle et pyrrolyle.

Par radical alkyle, alkényle ou alkynyle, linéaire ou ramifié, renfermant chacun au plus 8 atomes de carbone on entend de préférence le radical choisi parmi méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthyl pentyle, 3,3-diméthyl pentyle, 3-éthylpentyle, n-octyle, 2,2-diméthylhexyle, 3,3-diméthylhexyle, 3-méthyl 3-éthylpentyle, vinyle, propényle, isopropényle, allyle, 2-méthylallyle, butényle, isobutényle, éthynyle, propynyle, propargyle, butynyle et isobutynyle et tout particulièrement le radical méthyle, éthyle, vinyle, propényle, éthynyle et propynyle.

Par radical aryle renfermant de 6 à 14 atomes de carbone et aralkyle renfermant de 7 à 15 atomes de carbone et éventuellement substitué, on entend de préférence un radical phényle ou benzyle éventuellement substitué par un atome d'halogène choisi parmi fluor, chlore, brome et iode, un radical alkyle ayant de 1 à 8 atomes de carbone tel que méthyle, éthyle, propyle, isopropyle, butyle, isobutyle, tert-butyle, un radical alkoxy ayant de 1 à 8 atomes de carbone tel que méthoxy, éthoxy, propyloxy, isopropyloxy, butyloxy, un radical alkylthio ayant de 1 à 8 atomes de carbone tel que méthylthio, éthylthio, propylthio, isopropylthio, butylthio, un radical amino, alkylamino ayant de 1 à 8 atomes de

carbone tel que méthylamino ou éthylamino, dialkylamino ayant de 2 à 16 atomes de carbone tel que diméthylamino, diéthylamino, méthyléthylamino, chacun de ces radicaux dialkylamino étant éventuellement sous forme oxydée, un radical aminoalkyle ayant de 1 à 8 atomes de carbone tel que aminométhyle ou aminoéthyle, un radical dialkylaminoalkyle ayant de 3 à 16 atomes de carbone tel que diméthylamino méthyle ou éthyle, un radical dialkylaminoalkyloxy ayant de 3 à 16 atomes de carbone tel que en particulier le radical diméthylamino éthyloxy, un groupement hydroxyle, un groupement carboxy libre, estérifié tel que alkoxy carbonyle par exemple méthoxy carbonyle ou éthoxy carbonyle ou salifié par un atome de sodium ou de potassium, un radical cyano, un radical trifluorométhyle, un radical nitro, un radical formyle, un radical carbamoyle, un groupement acyle tel que acétyle, propionyle, butyryle ou benzoyle, acyloxy ayant au plus 12 atomes de carbone tel que acétoxy ou un groupement de formule :

$-O-CO-(CH_2)_mCO_2H$ dans lequel m est un entier qui va de 1 à 5, un radical alcényle tel que vinyle ou propényle, un radical alcynyle tel que éthynyle ou propynyle, un radical aryle tel que phényle, furyle, thiényle ou aralkyle tel que benzyle.

L'expression "aryle éventuellement substitué" indique qu'un ou plusieurs substituants, identiques ou différents, peuvent être présents en position ortho, méta ou para.

**[0012]** Lorsque $R_1$ et $R_2$ sont des radicaux alkyle, alkényle ou alkynyle substitués, il s'agit des substituants tels que définis plus haut.

L'invention s'étend naturellement aux sels des composés de formule (I), comme par exemple les sels formés, lorsque les composés de formule (I) comportent une fonction amino, avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcanesulfoniques tels que les acides méthane ou éthanesulfoniques, arènesulfoniques, tels que les acides benzène ou paratoluène sulfonique et arylcarboxylique, ou lorsque les composés de formule (I) comportent une fonction acide, avec les sels des métaux alcalins ou alcalino terreux ou d'ammonium éventuellement substitué.

L'invention a particulièrement pour objet à titre de médicaments les composés de formule (I) telle que définie précédemment répondant à la formule générale (I') :

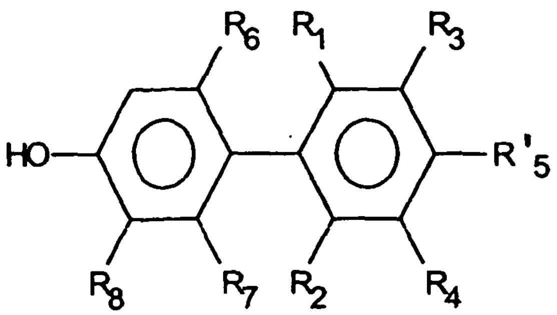

(I')

dans laquelle

$R'_5$ représente un groupement $CH_2OH$ et $R_1$, $R_2$ sont tels que définis précédemment, et $R_3$, $R_4$, $R_6$, $R_7$ et $R_8$ sont des atomes d'hydrogène, étant entendu que $R_1$ et $R_2$ ne peuvent simultanément représenter un atome d'hydrogène,

ainsi que les sels d'addition avec les acides et les bases pharmaceutiquement acceptables.

L'invention a plus particulièrement pour objet à titre de médicaments les composés de formule générale (I') telle que définie précédemment, dans laquelle $R_1$, $R_2$, $R_3$, $R_4$, $R_6$, $R_7$ et $R_8$ sont tels que définis précédemment, ainsi que les sels d'addition avec les acides ou les bases pharmaceutiquement acceptables.

L'invention a tout particulièrement pour objet à titre de médicaments les composés de formule (I') telle que définie précédemment dans laquelle $R'_5$ représente un radical $CH_2OH$ et $R_1$, $R_2$, $R_3$, $R_4$, $R_6$, $R_7$ et $R_8$ ont la même signification que précédemment ainsi que les sels d'addition avec les acides et les bases pharmaceutiquement acceptables.

$C(CH_3)_2$, $CH_2CH_2$, $CH_2CH_2CH_2$ ou $CH_2CH_2CH_2CH_2$

L'invention a plus particulièrement pour objet à titre de médicaments les composés de formule (I') telle que définie précédemment dans laquelle $R_6$, $R_7$ et $R_8$ sont des atomes d'hydrogène.

L'invention a plus particulièrement pour objet à titre de médicaments les composés de formule (I') telle que définie précédemment dans laquelle $R_6$ et $R_7$ sont des atomes d'hydrogène et $R_8$ est un groupement benzyle.

L'invention a tout particulièrement pour objet à titre de médicaments les composés de formule (I') telle que définie précédemment dans laquelle $R_1$ et $R_2$ identiques ou différents sont des atomes d'halogènes et $R_3$, $R_4$, $R_6$, $R_7$ et $R_8$ sont des atomes d'hydrogène.

L'invention a encore particulièrement pour objet à titre de médicaments les composés de formule (I) telle que définie précédemment répondant à la formule générale (I") :

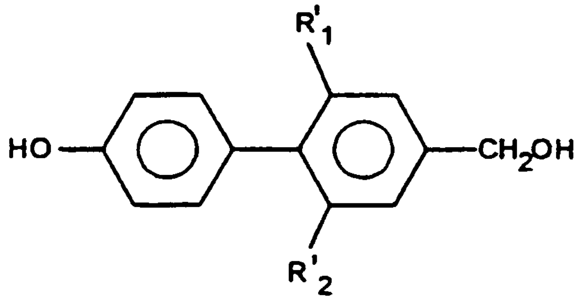

(I")

dans laquelle $R'_1$ représente un groupement aryle renfermant de 6 à 14 atomes de carbone et éventuellement substitué, $R'_2$ représente un atome d'halogène un radical nitro ou un radical amino, ainsi que les sels d'addition avec les acides et les bases pharmaceutiquement acceptables.

L'invention a tout particulièrement pour objet à titre de médicaments les composés de formule (I) telle que définie précédemment répondant à la formule générale (I") telle que définie ci-dessus dans laquelle $R'_1$ représente un radical phényle substitué par un groupement dialkylaminoalkyloxy ayant de 3 à 16 atomes de carbone et plus particulièrement le radical diméthylaminoéthyloxy,
ainsi que les sels d'addition avec les acides et les bases pharmaceutiquement acceptables.

L'invention a plus précisément pour objet à titre de médicaments les composés de formule (I) telle que définie précédemment dont les noms suivent :

- 2,6-dibromo-4'-hydroxy-(1,1'-biphényl)-4-mëthanol,
- 2,6-dichloro-4'-hydroxy-(1,1'-biphényl)-4-méthanol,
- 2,6-dinitro-4'-hydroxy-(1,1'-biphényl)-4-méthanol,
- 2-chloro-4'-hydroxy-6-(1-méthyléthyl)-(1,1'-biphényl)-4-méthanol,
- 2-chloro-4'-hydroxy-6-trifluorométhyl-(1,1'-biphényl)-4-méthanol,
- 2,6-dichloro-4'-hydroxy-5'-(phénylméthyl)-(1,1'-biphényl)-4-méthanol,
- 2-bromo 6-[[4-[2-(diméthylamino) éthoxy] phényl] hydroxyméthyl] 4'-hydroxy (1,1'-biphényl) 4-méthanol,
- 6'-bromo 4-[2-(diméthylamino) éthoxy] 4"-hydroxy (1,1':2',1"-terphényl) 4'-méthanol,
- 4-[2-(diméthylamino) éthoxy] 4"-hydroxy 6'-nitro (1,1':2',1"-terphényl) 4'-méthanol,
- 6'-chloro 4,4"-dihydroxy (1,1':2',1"-terphényl) 4'-méthanol.

La demanderesse a mis en évidence que les composés de formule (I) ainsi que leurs sels d'addition avec les acides et les bases pharmaceutiquement acceptables sont des produits particulièrement intéressants du point de vue pharmacologique. Ce sont des ligands originaux du récepteur estrogène.

A ce titre, les produits de formule (I) peuvent être utilisés, dans le traitement des troubles liés à une hypo folliculinie, par exemple, les aménorrhées, les dysménorrhées, les avortements répétés, les troubles prémenstruels, dans le traitement de certaines pathologies estrogéno-dépendantes telles que les adénomes ou carcinomes prostatiques, les carcinomes mammaires et ses métastases ou dans le traitement des tumeurs bénignes du sein, en tant qu'anti-utérotrophique ainsi que dans le traitement substitutif des symptômes liés à la ménopause et en particulier de l'ostéoporose.

L'invention s'étend aux compositions pharmaceutiques renfermant comme principe actif au moins un médicament tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de

suppositoires, d'ovules, de préparation injectables, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie en fonction de l'affection à traiter et de la voie d'administration : elle peut varier par exemple de 1 mg à 100 mg par jour chez l'adulte par voie orale.

Certains des produits de formule (I) sont nouveaux et sont donc un objet de la présente invention.

D'autres sont connus.

C'est ainsi que l'invention a pour objet l'application, à titre de médicament, d'une part des produits connus de formule générale (I) et d'autre part des produits nouveaux de formule générale (I).

L'invention a ainsi également pour objet les composés nouveaux de formule générale (I) répondant à la formule (I') telle que décrite précédemment, ainsi que leurs sels d'addition avec les acides et les bases.

L'invention a tout spécialement pour objet les composés de formule générale (I) telle que définie précédemment listés plus haut.

L'invention a encore particulièrement pour objet les composés nouveaux de formule (I) telle que définie précédemment répondant à la formule générale (I") telle que définie plus haut ainsi que leurs sels d'addition avec les acides et les bases.

L'invention a également pour objet un procédé de préparation des produits de formule (I') telle que définie ci-dessus caractérisé en ce que l'on soumet un produit de formule (II) :

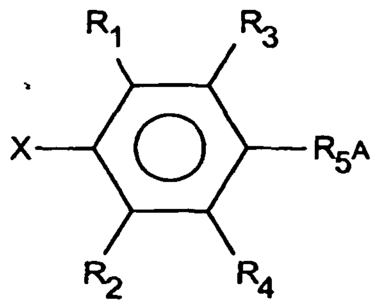

(II)

dans laquelle $R_1$, $R_2$, $R_3$ et $R_4$ sont tels que définis précédemment et $R_{5A}$ possède les valeurs de $R'_5$ telles que définies précédemment ainsi que les valeurs hydrogène ou $-CO_2H$ estérifié ou non estérifié et X représente un atome d'hydrogène, d'halogène ou un groupement $OSO_2CF_3$, à l'action, en présence d'un catalyseur, d'un produit de formule (III) :

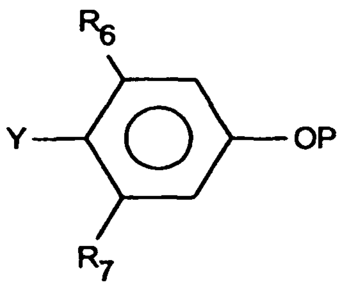

(III)

dans laquelle $R_6$ et $R_7$ sont tels que définis précédemment, Y représente un atome d'hydrogène, d'halogène, un groupement $B(OH)_2$ ou un groupement $Sn(R)_3$, dans lequel R représente un groupement alkyle renfermant de 1 à 8 atomes de carbone, et P représente un groupement protecteur, pour obtenir un produit de formule (IV):

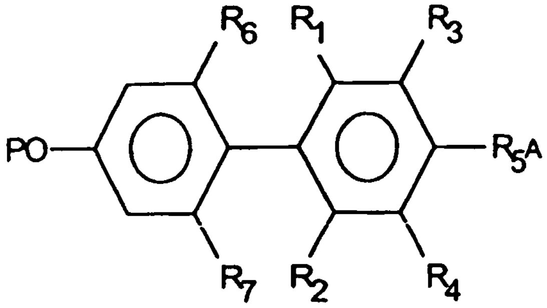

(IV)

dans laquelle P, $R_1$, $R_2$, $R_3$, $R_4$, $R_{5A}$, $R_6$ et $R_7$ ont la même signification que précédemment, produit de formule (IV) que si désiré ou si nécessaire l'on soumet dans un ordre approprié à l'une ou, le cas échéant, plusieurs des réactions suivantes afin d'obtenir le produit de formule (I') :

- déprotection du phénol,
- débenzylation puis réarrangement de façon à obtenir un produit de formule (I) avec $R_8$=benzyle,
- réduction totale ou partielle des groupements $NO_2$ que peuvent représenter $R_1$ ou $R_2$ en $NH_2$,
- substitution de $NH_2$ que peuvent représenter $R_1$, $R_2$, $R_3$ ou $R_4$ par Br ou par I,
- réaction de formylation lorsque $R_{5A}$ représente un atome d'hydrogène,
- réduction de la fonction $-CO_2H$ estérifiée que peut représenter $R_{5A}$,
- saponification,
- réduction du groupement -CHO que peut représenter $R_{5A}$ en un groupement $-CH_2OH$,

et salification par un acide ou une base.

La formation des biphényles de formule (IV) par couplage du composé aromatique de formule (II) avec le composé aromatique de formule (III) s'effectue
soit en présence d'un catalyseur choisi parmi les dérivés du palladium dans le cas où:

(a) Y représente un groupement $B(OH)_2$ ou $Sn(R)_3$ et X représente un groupement $OSO_2CF_3$, un atome de brome ou un atome d'iode,
et ainsi peut s'effectuer dans les conditions décrites dans les articles suivants lorsque Y représente un groupement $B(OH)_2$ :

- A. Huth, I. Beetz and I. Schumann Tetrahedron (1989) 45 6679 : Conditions: $Na_2CO_3$ 2M/$Pd(P\Phi_3)_4$/Toluène/LiCl/EtOH/Δ
- J. K. Stille et al. Ang. Chem. Int. Ed. (1986) 25 508 : Conditions : $Pd(P\Phi_3)_4$/LiCl/Dioxane/Δ
- T. Oh-e, N. Migawa and A. Suzuki J. Org. Chem. (1993) 58 2201-2208 : Conditions : $K_3PO_4$/KBr/$Pd(P\Phi_3)_4$/Dioxane/Δ
- Suzuki et al., Synlett (1992) 208
Conditions : $Pd(P\Phi_3)_4$/$Ba(OH)_2$/DMEaq ;
ou bien lorsque Y représente un groupement $SnBu_3$, dans les conditions décrites dans les articles suivants :
- J. K. Stille et al, J. Am. Chem. Soc. (1987) 5478-5480 ou par V. Farina, J. Org. Chem. (1993) 58 5434 ;

soit en présence de cuivre dans le cas où :
(b) Y représente un atome d'iode et X représente un atome de chlore,
(c) Y représente un atome de chlore et X représente un atome d'iode,
et ainsi peut s'effectuer dans les conditions décrites dans l'article suivant :

- P. E. Fanta Chem. Rev. (1964) 38 139 ou synthesis (1974) 9 : Conditions : Cu/DMF/120°C ;

soit en présence d'une base forte et de $ZnCl_2$ puis d'un catalyseur choisi parmi les dérivés du palladium dans le cas où :
(d) Y représente un atome de brome et X représente un atome d'hydrogène,
(e) Y représente un atome d'hydrogène et X représente un atome de brome,

et ainsi peut s'effectuer dans les conditions suivantes :

1) nBuli/THF/-768°C
2) $ZnCl_2$
3) $ArBr/Pd(P\Phi_3)_4/\Delta$
4) HCl/MeOH.

Les réactions d'orthométallation sont décrites par exemple dans les documents suivants :

T. KRESS Synthesis (1983) 803,
N. IWAO J. Org. Chem. (1990) 55 3623.

Par ailleurs, la réaction d'échange avec $ZnCl_2$ suivie d'une réaction de couplage a été décrite par E. Negishi dans J. Org. Chem. (1977) 42 182.

Le groupement protecteur P représente de préférence un radical alkyle renfermant de 1 à 4 atomes de carbone, un groupement benzyle, un groupement $R_CR_DR_ESi$, dans lequel $R_C$, $R_D$ et $R_E$ identiques ou différents, indépendamment l'un de l'autre représentent chacun un radical alkyl renfermant de 1 à 4 atomes de carbone ou un groupement phényle. Il s'agit tout particulièrement des groupements $Si(Me)_2C(Me)_3$ ou $-Si(Ph)_2C(Me)_3$.

Les réactions de déprotection sont les méthodes de déprotection classiques connues de l'homme du métier. Une revue assez complète se trouve dans l'ouvrage suivant :

Protective groups in organic synthesis T.W greene, John Wiley & sons (1981).

A titre d'exemple les réactions de déprotection lorsque P est un radical méthyle peuvent s'effectuer par action de tribromoborane dans le dichlorométhane ou d'acide chlorhydrique dans la pyridine, les réactions de déprotection lorsque P est un groupement benzyle peuvent s'effectuer par action d'hydrogène en présence de palladium sur charbon dans l'acétate d'éthyle, par action d'acide trifluoroacétique ou par action d'iodure de triméthylsilyle. Les réactions de déprotection lorsque P est un groupement tertbutyldiphénylsilyle peuvent s'effectuer par action de fluorure de tétrabutyl d'ammonium (TBAF) en solution dans le tétrahydrofuranne.

Lors de la réaction de débenzylation par action d'acide trifluoroacétique, on peut obtenir un réarrangement et formation d'un dérivé déprotégé avec $R_8$ = benzyle.

La réaction de réduction du radical $NO_2$ que peuvent représenter $R_1$ ou $R_2$ en radical $NH_2$ peut s'effectuer par action de dichlorure d'étain dans l'éthanol au reflux et la réaction de monoréduction s'effectue de préférence par action du cyclohexène en présence du dihydroxyde de palladium dans l'éthanol ou le tétrahydrofuranne au reflux.

La réaction de substitution de $NH_2$ que peuvent représenter $R_1$ ou $R_2$ par Br s'effectue de préférence par action d'acide bromhydrique en présence de nitrite de sodium et de bromure de cuivre dans l'eau à 0°C ou par action de tribromométhane en présence de terbutylnitrite.

La réaction de substitution de $NH_2$ par l'iode s'effectue de préférence par action d'iodure de potassium en présence de nitrite de sodium et d'acide sulfurique ou par action d'iode en présence de terbutylnitrite.

La réaction de formylation lorsque $R_{5A}$ est un atome d'hydrogène peut s'effectuer en présence de diméthylformamide et d'une base forte telle que le n-butyllithium puis hydrolyse avec un acide minéral tel que l'acide chlorhydrique.

La réaction de réduction de la fonction $-CO_2H$ estérifiée que peut représenter $R_{5A}$ afin d'obtenir l'alcool correspondant s'effectue par exemple par action d'aluminohydrure de lithium dans le tétrahydrofuranne.

La réaction de réduction du groupement -CHO que peut représenter $R_{5A}$ en un groupement $-CH_2OH$ s'effectue par exemple par action du borohydrure de sodium dans le méthanol à 0°C ou par action d'hydrogène en présence de palladium sur charbon dans l'acétate d'éthyle.

La salification peut être effectuée dans des conditions usuelles. On opère par exemple en présence de soude éthanolique. On peut également utiliser un sel de sodium tel que le carbonate ou le carbonate acide de sodium ou de potassium.

De même, la salification par un acide est réalisée dans les conditions usuelles. On opère par exemple avec l'acide chlorhydrique, par exemple en solution éthérée.

L'invention a notamment pour objet un procédé de préparation des produits de formule (I") telle que définie ci-dessus caractérisé en ce l'on soumet un composé de formule (VII) :

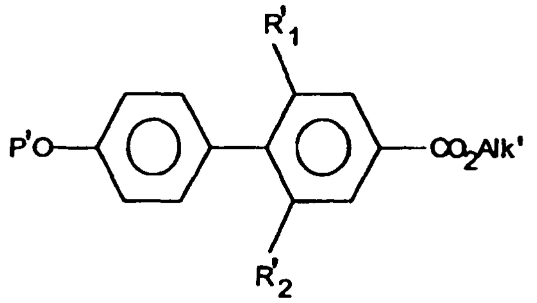

(VII)

dans lequel Alk' et P' identiques ou différents, représentent chacun un radical alkyle renfermant de 1 à 4 atomes de carbone et $R'_1$ et $R'_2$ sont tels que définis précédemment, à l'action d'un réactif de déprotection et de réduction de la fonction ester afin d'obtenir le produit de formule (I") puis, si désiré, à l'action d'une base ou d'un acide pour obtenir les sels correspondants.

Ce produit de formule (VII) correspond aux produits de formule (IV) dans lesquels P représente un radical alkyle renfermant de 1 à 4 atomes de carbone, $R_2$, $R_3$, $R_4$, $R_6$, $R_7$ et $R_8$ sont des atomes d'hydrogène, $R_1$ représente un radical aryle éventuellement substitué et $R_{5A}$ représente un groupement $-CO_2H$ estérifié.

Un procédé de formation des produits de formule (VII), avec $R'_1$ représentant un groupement Ph-OH, P et Alk représentant un méthyle, est décrit dans la référence suivante : J. Med. Chem. (1989) 32 1814-1820.

Ce procédé peut ainsi être étendu par analogie à tous les produits de formule (V) avec $R'_1$ représentant un groupement aryle renfermant de 6 à 14 atomes de carbone éventuellement substitués.

Ainsi on soumet un composé de formule (V) :

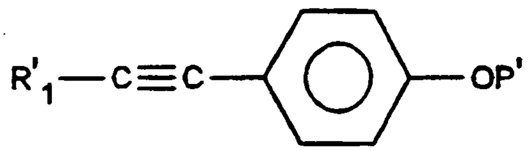

(V)

dans laquelle $R'_1$ représente un groupement aryle éventuellement substitué et P' représente un radical alkyle renfermant de 1 à 4 atomes de carbone, à l'action d'un composé de formule (VI) :

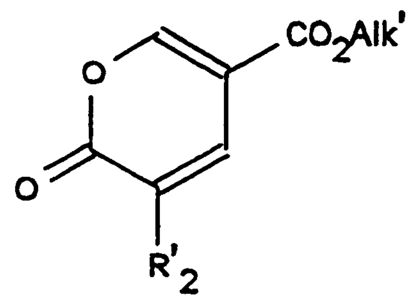

(VI)

dans laquelle Alk' représente un radical alkyle renfermant de 1 à 4 atomes de carbone et $R'_2$ est tel que défini précédemment, afin d'obtenir un composé de formule (VII).

L'action de l'alkylcoumarate de formule (VI) sur le composé de formule (V) afin d'obtenir le composé de formule (VII) s'effectue préférentiellement sous pression d'azote à une température de 250°C dans le toluène. Cette réaction est décrite dans la référence suivante : J. Med. Chem. (1989) 32 1814-1820.

Les produits de formule (V) sont connus ou sont aisément accessible à l'homme du métier en appliquant les principes généraux de fonctionnalisation des composés aromatiques. On peut citer à titre d'exemples quelques références :

WO9309079 ($R'_1$=Ph et P'=Me), J. Organomet. Chem. 395(2), 277-9 ($R'_1$=Ph-$OCH_2CH_2NMe_2$ et P'=Me,).

Les produits de formule (VI) avec $R'_2$ = H, sont également connus. On peut citer la référence suivante : J. Med. Chem. (1989) 32 1814-1820.

Les produits de formule (VI) avec $R'_2$ différent de H, sont connus ou aisément accessibles à l'homme du métier. Par exemple, les produits de formule (VI) avec $R'_2$ = Cl sont formés par action de N-chlorosuccinimide sur le produit de formule (VI) avec $R'_2$ = H.

Les produits de formule générale (II) sont en général connus ou sont accessibles à l'homme du métier en appliquant les principes généraux de fonctionnalisation des composés aromatiques.

Les produits de formule (II) avec X=0Tf (triflates) sont obtenus à partir des alcools correspondants de formule générale (II') :

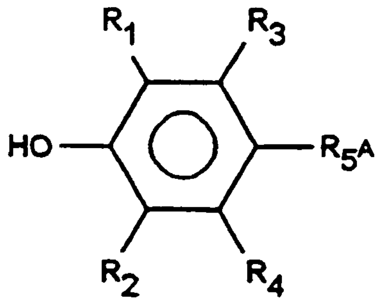

(II')

par action d'anhydride triflique dans la pyridine à 0°C selon la méthode décrite par Scott W.J., Stille J.K., J. Am. Chem. Soc. (1986) 108 3033.

Les produits de formule générale (II') sont quant à eux en général connus ou sont accessibles par l'homme du métier en appliquant les principes généraux de la chimie des composés aromatiques. On peut citer entre autre la référence suivante : RODD'S CHEMISTRY OF CARBON COMPOUNDS Vol III Aromatic compounds Ed. M.F. ANSELL Elsevier Scientific Publishing Company (1981).

Les produits de formule (II) avec X= I (produits iodés) peuvent être obtenus par orthométallation à partir produits aromatiques non iodés correspondants de formule générale (II") :

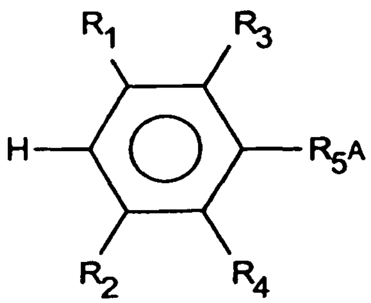

(II")

notamment par action de N-iodosuccinimide ou d'iode en présence d'une base forte telle que le n-Butyllithium dans le tétrahydrofuranne à -78 C.

Les produits de formule générale (II") sont quant à eux en général connus ou sont accessibles par l'homme du métier en appliquant les principes généraux de la chimie des composés aromatiques.

Dans le cas où $R_{5A}$ est un groupement -$CO_2H$, il faudra prévoir les protections adéquates connues de l'homme du métier notamment lors de la préparation des produits de formule générale (II) à partir des produits de formules générales (II') et (II"). A titre d'exemple, le groupement $CO_2H$ peut être estérifié, le groupement formyle ou acyle peut être protégé sous la forme d'un acétal tel que le dioxolanne par action du glycol en présence d'acide paratoluène sulfonique, le groupement hydroxyle peut être protégé sous la forme d'un groupement tétrahydropyrranyloxy (OTHP) par action du dihydropyrrane.

Les produits de formule (III) sont connus ou accessibles à partir de parabromophénol protégé ou de parabromoanisole par les méthodes suivantes :

Les produits de formule (III) avec Y représentant le groupement $B(OH)_2$ et P représentant un radical méthyle peuvent être obtenus d'abord par action du parabromoanisole avec du Mg en tournures dans l'éther diéthylique anhydre au reflux, puis par action du triéthylborate dans l'éther diéthylique anhydre à -70 C, puis d'une hydrolyse avec un acide minéral fort tel que l'acide sulfurique.

Les produits de formule (III) avec Y représentant le groupement $B(OH)_2$ peuvent également être obtenus par action du parabromophénol protégé par un groupement protecteur P tel que benzyle ou terbutyldiphényle silyle, avec du triéthylborate en présence de n-butyllithium dans le tétrahydrofuranne à -78°C suivi d'une hydrolyse avec un acide minéral fort tel que l'acide sulfurique ou avec de l'eau.

Les produits de formule (III) avec Y représentant le groupement $SnBu_3$ peuvent être obtenus par action du parabromophénol protégé par un groupement protecteur P tel que tertbutyldiphénylsilyle, avec du chlorure de tributyl étain en présence de n-butyllithium dans le tétrahydrofuranne à -78 C.

Les produits de formule (III) avec Y représentant un atome d'iode et P représentant un groupement benzyle ou terbutyldiphénylsilyle peut être obtenu par action du para-iodophénol avec un groupement protecteur tel que défini plus haut.

Les exemples suivants illustrent l'invention sans toutefois la limiter.

**PREPARATION 1 : Acide-(4-méthoxyphényl)-boronique**

On ajoute goutte à goutte au reflux 100 ml d'une solution de 10 g de p-bromoanisole dans l'éther diéthylique anhydre à une suspension, sous gaz inerte, de 1,3 g de magnésium en tournure dans 5 ml d'éther diéthylique anhydre, et laisse le mélange au reflux pendant 2 heures. Le milieu réactionnel est ensuite versé dans une solution de 9,02 ml de triéthylborate dans 60 ml d'éther anhydre refroidie à -70°C. Après agitation durant 1 heure à -70°C, puis 1 heure à température ambiante, on verse la solution dans un mélange comprenant 11 ml d'acide sulfurique et 50 g de glace et d'eau et agite pendant 1 heure. On extrait la phase organique avec 100 ml d'une solution aqueuse saturée en bicarbonate de soude, réunit les phases aqueuses, les réacidifie avec de l'acide chlorhydrique 6N, extrait avec de l'éther, séche et évapore sous pression réduite. On obtient 3,9 g du produit attendu. Spectre I.R. : (Nujol)
Absorption complexe région OH/NH, 1609, 1573 et 1518 $cm^{-1}$ RMN (DMSO-d6, 300MHz)

| | |
|---|---|
| 3,76 s | $OC\underline{H}_3$ |
| 6,88 d J=9Hz | $H_3$ et $H_5$ |
| 7,78 d J=9Hz | $H_2$ et $H_6$ |
| 7,86 | $B(OH)_2$ |

**PREPARATION 2 : Acide-[4-(phénylméthoxy)phényl]-boronique**

Stade A : 1-bromo-4-(phénylméthoxy)-benzène

On ajoute, à 0°C, 15,26 g d'hydrure de sodium à 50 % dans l'huile à une solution sous gaz inerte de 50 g de parabromophénol dans 320 ml de diméthylformamide, agite pendant 30 minute à 0°C, puis ajoute 37,7 ml de bromure de benzyle. On agite pendant 2 heures 30 en laissant remonter la température à 20°C, puis coule le mélange réactionnel sur de l'eau glacée, filtre le précipité, et séche. On obtient 73,35 g de produit attendu. Rf : 0,85 (chromatographie sur couche mince, support : silice, éluant : cyclohexane/acétate d'éthyle 7/3).
Spectre I.R. : ($CRCl_3$)
Absence d'OH
Aromatique 1592, 1580 et 1488 $cm^{-1}$

Stade B : Acide [4-(phénylméthoxy)phényl]-boronique

On ajoute, goutte à goutte, sous gaz inerte et à -78°C, 143 ml d'une solution de n-Butyllithium à 47,08 g du produit obtenu au stade A dans 375 ml de tétrahydrofuranne, agite pendant 1 heure, puis ajoute 36,5 ml de triéthylborate. On agite 14 heures, en laissant remonter la température à 20°C, et on hydrolyse le milieu réactionnel au moyen d'une solution d'eau glacée contenant 45 ml d'acide sulfurique concentré, pendant 1 heure à 20°C. La phase aqueuse est extraite avec de l'acétate d'éthyle, les phases organiques sont lavées par de la soude 2N et la phase aqueuse est acidifiée à pH=1 à l'aide d'une solution d'acide chlorhydrique 1N afin de précipiter l'acide boronique. Après filtration et

séchage du précipité on obtient 28,54 g du produit attendu.
Rf : 0,16 cyclohexane/acétate d'éthyle 7/3)
Spectre I.R. : (Nujol)

| | |
|---|---|
| Absorption générale région OH/NH | 3650, 3615, 3510 et 3420 $cm^{-1}$ |
| Aromatique | 1605, 1570 et 1510 $cm^{-1}$ |
| B-O | 1410, 1340 $cm^{-1}$ |

**PREPARATION 3 : Acide[4-[[(1,1-diméthyléthyl)diphénylsilyl] oxy]phényl]-boronique**

Stade A : 1-Bromo-4-[[(1,1-diméthyléthyl)diphénylsilyl]oxy] benzène

Sous atmosphère inerte et à température ambiante on ajoute à 80,89 g de parabromophénol, 400 ml de diméthylformamide, 31,18 g d'imidazole et 125,89 g de 1,1-diméthyl-éthyl-diphényl-chlorosilane puis on agite la solution obtenue pendant 2 heures. On verse dans 2 litres d'eau, observe une précipitation, solubilise le solide avec de l'acétate d'éthyle et extrait la phase aqueuse avec de l'acétate d'éthyle, sèche les phases organiques réunies et évapore sous pression réduite jusqu'à obtention d'une huile. On ajoute du pentane et observe une cristallisation. Après filtration et séchage du précipité on obtient 179,24 g du produit attendu. Rf : 0,53 (chromatographie sur couche mince, support : silice, éluant Cyclohexane/AcOEt 95/5).
Point de fusion : 56°C
RMN ($CDCl_3$, 300MHz)

| | |
|---|---|
| 1,09 s | Si-tBu |
| 6,63 m | $H_3$, $H_5$ |
| 7,17 m | $H_2$, $H_6$ |
| 7,69 dd | 4H pour SiΦ2 |
| 7,4 | 6H pour SiΦ2 |

Stade B : Acide [4-[[(1,1-diméthyléthyl)diphénylsilyl]oxy] phényl]-boronique

A une solution de 30 g du produit du stade précédent dans 100 ml de tétrahydrofuranne anhydre, on ajoute, goutte à goutte, à -78°C et sous gaz inerte, 60 ml d'une solution de n-butyl-lithium puis après agitation durant 30 minutes à -78°C, 9,95 ml de triméthylborate. Après 2 heures trente d'agitation, en laissant revenir à température ambiante, on additionne goutte à goutte 20 ml d'eau et agite pendant 72 heures. Après évaporation sous pression réduite du tétrahydrofuranne, on extrait la phase aqueuse à l'éther, sèche et concentre sous pression réduite jusqu'à obtention d'une huile (26,35 g) que l'on purifie par chromatographie filtrante sur silice avec le mélange hexane/acétate d'éthyle 1/1 pour obtenir 7,73 g du produit attendu sous forme de trimère et de monomère.
IR ($CHCl_3$)

| | |
|---|---|
| O-Si | 915 et 1255 $cm^{-1}$ |
| B-O | 1350 et 1370 $cm^{-1}$ |

Aromatiques 1515, 1570 et 1602 $cm^{-1}$
RMN ($CDCl_3$)

| | |
|---|---|
| 1,11 | tBu |
| 6,81 et 7,88 | Ph-O |
| 7,3 à 7,5 (6H) et 7,72 (4H) | PhSi |

**PREPARATION 4 : [4-[[(1,1-diméthyléthyl)diphénylsilyl]oxy] phényl]-tributyl étain**

On ajoute, goutte à goutte, à -50°C et sous gaz inerte, 123,89 ml d'une solution de sec-butyl-lithium (1,13 M) à une solution de 50 g du produit du stade A de la préparation 3 dans 300 ml de tétrahydrofuranne anhydre, puis après agitation durant 1 heure à -50°C, ajoute 36,29 ml de chlorure de tributyl étain. Après 30 minutes d'agitation, en laissant

revenir à température ambiante, on verse le mélange réactionnel dans de l'eau glacée, extrait la phase aqueuse avec de l'acétate d'éthyle, sèche et évapore à sec sous pression réduite. On obtient 38,5 g du produit obtenu sous forme d'une huile ($T_{eb}$ : 230°C sous $10^{-2}$ mbar)
Rf : 0,36 cyclohexane.
IR ($CHCl_3$)
Aromatique 1569, 1583, 1510, 1493 $cm^{-1}$

**PREPARATION 5 : 1-iodo-4-[[diméthyl(1,1-diméthyléthyl)silyl] oxy]-benzène.**

On ajoute à température ambiante et sous atmosphère inerte 387 mg d'imidazole et 411 mg de chlorure de tertbutyl-diméthylsilyle à 500 mg de para-iodo-phénol dans 4 ml de diméthylformamide et on maintient le mélange sous agitation pendant 15 heures à température ambiante puis pendant 1 heure à 40°C. On verse le milieu réactionnel dans de l'eau, extrait la phase aqueuse avec du dichlorométhane. La phase organique est ensuite séchée et évaporée sous pression réduite. On obtient ainsi 636 mg du produit attendu.
Rf = 0,82 cyclohexane/acétate d'éthyle 9/1).
RMN ($CDCl_3$) 200MHz

| | | |
|---|---|---|
| 0,1 p.p.m | (s) | $CH_3$ |
| 0,9 p.p.m | (s) | tBu |
| 6,55 p.p.m | (d) à 7,5 p.p.m (d) : les 4H aromatiques | |

**PREPARATION 6 : 1-iodo-4-(phénylméthoxy)-beazène**

On ajoute à 0°C et sous atmosphère inerte 2,4 g d'hydrure de sodium à 50 % dans l'huile à 10 g de para-iodo-phénol dans 150 ml de diméthylformamide, maintient le mélange sous agitation pendant 30 minutes et ajoute 5,9 ml de bromure de benzyle. Après 30 minutes d'agitation en laissant remonter la température à l'ambiante, on verse le milieu réactionnel sur de la glace et observe une précipitation du produit.
Après séchage, on obtient 14,7 g du produit attendu.
Rf = 0,67 cyclohexane/acétate d'éthyle 9/1).
RMN ($CDCl_3$) 200MHz

| | | | |
|---|---|---|---|
| 7,35 | (m) | | H aromatiques du benzyle |
| 7,5 | (d) | 2H | $H_2$ et $H_6$ |
| 6,7 | (d) | 2H | $H_3$ et $H_5$ |
| 5 | (s) | | $CH_2Ph$ |

**PREPARATION 7 : 3,5-dibromo-4-[[(trifluorométhyl)sulfonyl]-oxy]-benzaldéhyde**

On ajoute, sous gaz inerte et à 0°C, 14,73 ml d'anhydride triflique à 19,0 g de 3,5-dibromo-4-hydroxy-benzaldéhyde dans 100 ml de pyridine. On agite pendant 1 heure en laissant la température évoluer à l'ambiante, verse dans de l'eau et extrait la phase aqueuse avec 3 fois 150 ml de dichlorométhane. Les phases organiques sont séchées et évaporées à sec sous pression réduite. On obtient 23,83 g de produit brut que l'on purifie par chromatographie filtrante en utilisant le mélange cyclohexane/acétate d'éthyle 1/1 comme éluant. On recueille ainsi 23,83 g de produit attendu
Rf : 0,71, cyclohexane/acétate d'éthyle 1/1.
On opère de façon équivalente pour la préparation des triflates de formule générale II suivants

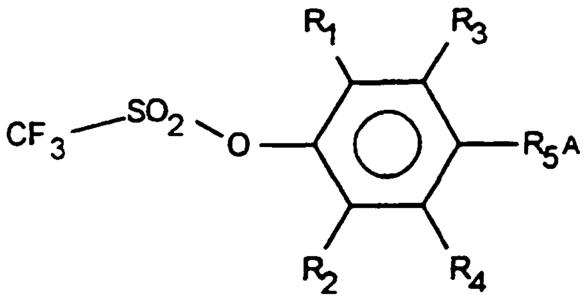

| Préparation | $R_1$ | $R_3$ | $R_2$ | $R_4$ | $R_{5a}$ | Rf | $C_6H_{12}$/AcOEt |
|---|---|---|---|---|---|---|---|
| 8 | Me | H | Me | H | CHO | 0,34 | 9/1 |
| 9 | H | Me | iPr | H | $COCH_3$ | 0,64 | 7/3 |
| 10 | iPr | H | Cl | Me | $COCH_3$ | 0,44 | 7/3 |
| 11 | Cl | H | OMe | H | CHO | 0,29 | 8/2 |
| 12 | Cl | H | Cl | H | CHO | 0,53 | 7/3 |
| 13 | iPr | H | Cl | H | CHO | 0,35 | 9/1 |
| 14 | H | H | Cl | H | $CO_2Me$ | 0,74 | 7/3 |
| 15 | CH=CH-CH=CH | | Cl | H | CHO | 0,75 | 7/3 |
| 16 | Cl | H | $CF_3$ | H | CHO | 0,22 | 9/1 |

**<u>EXEMPLE 1</u> : 2,6-dibromo-4'-hydroxy-(1,1-biphényl)-4-carboxaldéhyde**

<u>Stade A</u> : Couplage

2,6-dibromo-4'-méthoxy-(1,1'-biphényl)-4-carboxaldéhyde

A 1,32 g du triflate obtenu à la préparation 7, on ajoute, sous gaz inerte, 50 ml de toluène, 20 ml d'éthanol, 6,6 ml de carbonate de sodium 2M, 534 mg d'acide (4-méthoxyphényl)-boronique obtenu comme à la préparation 1, 245 mg de LiCl, 112 mg Tétrakis(triphénylphosphine)-palladium(0) et on agite au reflux pendant 1 heure 30. Après dilution avec de l'acétate d'éthyle la phase organique est lavée avec de la soude 2N, puis avec une solution saturée en NaCl, séchée puis évaporée sous pression réduite. On purifie par chromatographie sur silice en éluant avec un mélange cyclohexane-acétate d'éthyle (98-2)et obtient 89 mg de produit attendu.
Rf = 0,68 cyclohexane/acétate d'éthyle (7/3))
R.M.N. ($CDCl_3$, 300MHz)

| | | | |
|---|---|---|---|
| 3,88 | (s) | | OC<u>H</u>$_3$ |
| 7,01-7,15 | | (AA'BB') | Ph-O- |
| 8,12 | | | $H_3$, $H_5$ |
| 9,94 | (s) | | CHO |

<u>Stade B</u> : Déprotection

2,6-dibromo-4'-hydroxy-(1,1'-biphényl)-4-carboxaldéhyde

A 1,2 g du produit préparé au stade précédent on ajoute sous gaz inerte, 15 ml de dichlorométhane puis, à -45°C, 0,75 ml de BBr3 et on agite 1 heure à -45°C puis 15 heures en laissant la température évoluer de -45°C à -22°C. Après hydrolyse par addition d'une solution saturée en acétate de sodium et extraction avec du dichlorométhane et de l'acétate d'éthyle, la phase organique est séchée et évaporée sous pression réduite. Le brut réactionnel est chromatographié sur silice en éluant avec un mélange cyclohexane-acétate d'éthyle (98-2). 471 mg du produit attendu sont ainsi recueillis.
Rf = 0,28 cyclohexane/acétate d'éthyle (8/2)
I.R. Nujol
Absorption OH/NH - 3450 $cm^{-1}$
C=O 1684 $cm^{-1}$
Aromatique 1611, 1589, 1534, 1518 $cm^{-1}$
RMN ($CDCl_3$, 300 MHz)
4,97 (s, OH) ; 6,95 et 7,20 (AA'BB', Ph-O) ; 8,12 (s) H3, H5 ; 9,94 (s, CHO).

**EXEMPLE 2 : 2,6-dibromo-4-hydroxy-(1,1'-biphényl)-4-méthanol**

On refroidit à 0°C 140 mg du produit préparé à l'exemple 1 dans 3 ml de méthanol, ajoute 15 mg de borohydrure de sodium et agite 1 heure à 0°C. Le méthanol est évaporé sous pression réduite et on ajoute de l'eau au résidu. La phase aqueuse est extraite avec de l'acétate d'éthyle, et la phase organique est évaporée sous pression réduite. Le résidu est ensuite chromatographié sur silice en éluant avec le mélange cyclohexane/acétate d'éthyle 9/1. On obtient 47 mg du produit attendu.
Rf = 0,67 cyclohexane/acétate d'éthyle (4/6)
I.R. Nujol
Absence de C=O.
Absorption générale OH/NH
Aromatique 1612, 1594, 1518 $cm^{-1}$
RMN ($CDCl_3$, 300 MHz)
1,83 (t, OH) ; 4,71 (d, $CH_2$) ; 4,95 (s, OH) ; 6,92-7,08 (AA'BB', Ph-O) ; 7,64 (s, H3, H5).

**EXEMPLE 3 : 2-chloro-4'-hydroxy-6-méthoxy-(1,1'-biphényl)-4-méthanol**

Stade A : Couplage

2-chloro-6-méthoxy-4'-phénylméthoxy-(1,1'-biphényl)-4-carboxaldéhyde

A une solution, sous atmosphère inerte, de 3 g de triflate obtenu à la préparation 11 dans 20 ml de dioxane, on ajoute 1,4 g de bromure de potassium, 3,7 g de K3PO4 mono-hydraté, 2,68 g puis 0,55 g d'acide boronique obtenu à la préparation 2, 0,62 g de Tétrakis(triphénylphosphine)-palladium(0) et on agite 12 heures à 110°C. Après filtration puis évaporation sous pression réduite, le mélange est chromatographié sur silice en éluant avec un mélange cyclohexane/acétate d'éthyle 9/1 puis 8/2. On obtient 762 mg du produit pur attendu.
Rf= 0,4 cyclohexane/acétate d'éthyle 8/2
RMN ($CDCl_3$ 250MHz)

| | | |
|---|---|---|
| 3,82 | (s) | $OC\underline{H}_3$ |
| 5,11 | (s) | $C\underline{H}_2Ph$ |
| 7,08 et 7,25 | AA'BB' | Ph-O |
| 7,37 et 7,6 | (2d) J=2,5 | $H_3$, $H_5$ |
| 7,3-7,5 | (m) | H du benzyle |
| 9,95 (s) | | $C\underline{H}O$ |

Stade B : déprotection et réduction

2-chloro-4'-hydroxy-6-méthoxy-(1,1'-biphényl)-4-méthanol

On mélange à température ambiante 200 mg du produit obtenu au stade précédent dans 5 ml d'éthanol avec 60 mg de palladium sur charbon à 9,5 %. On place le milieu sous atmosphère d'hydrogène et agite 3 heures à 20°C. Après filtration, on évapore sous pression réduite pour obtenir 265 mg d'une huile marron. Le mélange est purifié par chromatographie en éluant avec un mélange cyclohexane/acétate d'éthyle 8/2, puis 7/3 et enfin 1/1. On obtient 62 mg de produit attendu ainsi que 37 mg du produit réduit ($R_5$=méthyle).

Rf = 0,1 cyclohexane/acétate d'éthyle 7/3
RMN ($CDCl_3$ 250MHz)

| | | |
|---|---|---|
| 1,74 | (t) | $CH_2O\underline{H}$ |
| 4,71 | (d) | $C\underline{H}_2OH$ |
| 3,75 | (s) | $OC\underline{H}_3$ |
| 4 , 79 | (s) | $O\underline{H}$ |
| 6,9 et 7,16 | AA'BB' | Ph-O |
| 6,91 et 7,09 | 2d | $H_3$, $H_5$ |

**<u>EXEMPLE 4</u> : 2-chloro-4'-hydroxy-6-(1-méthyléthyl)-(1,1'-biphényl)-4-méthanol**

On procède de manière équivalente à l'exemple 3 stade A et B (couplage + déprotection) ainsi qu'à l'exemple 2 (réduction) à partir de 210,9 mg du triflate obtenu à la préparation 13 et on obtient 36 mg du produit attendu.
Rf=0,32 cyclohexane/acétate d'éthyle 7/3
RMN ($CDCl_3$ 300MHz)

| | | |
|---|---|---|
| 1,09 | (d) | $CH(C\underline{H}_3)_2$ |
| 2,81 | (sept) | $C\underline{H}(CH_3)_2$ |
| 4,60 | (s) | $C\underline{H}_2OH$ |
| 7,28 | (m) | $H_3$, $H_5$ |
| 6,88 et 6,94 | AA'BB' | Ph-O |

**<u>EXEMPLE 5</u> : 2-chloro-4'-hydroxy-(1,1'-biphényl)-4-méthanol**

<u>Stade A</u> : Couplage

2-chloro-4'-phénylméthyloxy-(1,1'-biphényl)-4-carboxylate de méthyle

On opère de manière équivalente à l'exemple 3 stade A à partir de 1,58 g de triflate obtenu à la préparation 14 et on obtient 0,96 g de produit attendu.
Rf=0,42 cyclohexane/acétate d'éthyle 90/10
RMN ($CDCl_3$ 300MHz)

| | | | |
|---|---|---|---|
| 3 , 95 | s | | $CO_2C\underline{H}_3$ |
| 5, 12 | s | | $PhC\underline{H}_2$ |
| 8,13 | d | | $H_3$ |
| 7 , 9 5 | dd | | $H_5$ |
| 7,30 7,50 | m | 6H | H du benzyle + $H_6$ |
| 7,04 et 7,41 | AA'BB' | | Ph-O |

<u>Stade B</u> : réduction de l'ester

2-chloro-4'-phénylméthyloxy-(1,1'-biphényl)-4-méthanol

On mélange pendant 2 heures, sous atmosphère inerte et à 0 C, 204,4 mg d'ester dans 10 ml de tétrahydrofuranne et 44 mg d'hydrure de lithium et d'aluminium, puis on coule le mélange dans une solution saturée en bicarbonate de sodium. Après extraction à l'acétate d'éthyle, séchage et évaporation sous pression réduite, on obtient 174 mg du produit attendu.
Rf = 0,17 cyclohexane/acétate d'éthyle 8/2.

Stade C : déprotection

2-chloro-4'-hydroxy-(1,1'-biphényl)-4-méthanol

On opère de manière équivalente à l'exemple 3 stade B à partir de 174 mg du dérivé benzylé de l'étape précédente et on obtient 55 mg du produit attendu et 37 % de l'analogue réduit ($R_5=CH_3$, Rf=0,53 cyclohexane/acétate d'éthyle 7/3)
Rf=0,18 cyclohexane/acétate d'éthyle 7/3
RMN DMSO 250MHz

| | | |
|---|---|---|
| 4,52 | s | C<u>H</u>$_2$OH |
| 5,33 | s | CH$_2$O<u>H</u> |
| 7,30 sl 2H et 7,44 s 1H | | $H_3$, $H_5$, $H_6$ |
| 6,84 d et 7,23 d | | Ph-O |
| 9,59 | s | Ph-O<u>H</u> |

**EXEMPLE 7 : 2-chloro-4'-hydroxy-6-(trifluorométhyl)-(1,1'-biphényl)-4-méthanol**

On procède de manière équivalente à l'exemple 3 à partir de 285 mg de triflate obtenu à la préparation 16 et on obtient 10,6 mg de produit attendu.
Rf=0,23 cyclohexane/acétate d'éthyle : 7/3
RMN (DMSO, 300 MHz)

| | | |
|---|---|---|
| 4,62 | d | C<u>H</u>$_2$OH |
| 5,54 | t | CH$_2$O<u>H</u> |
| 6,82 et 6,98 AA'BB' | | Ph-OH |
| 7,72 sl 7,76 sl | | $H_3$, $H_5$ |
| 9,62 | s | Ph-O<u>H</u> |

**EXEMPLE 8 : 3-chloro-4-(4-hydroxyphényl)-1-naphtalène-méthanol**

Stade A : Couplage

3-chloro-4-(4-phénylméthoxyphényl)-1-naphtalène-carboxaldéhyde.

On procède de manière équivalente à l'exemple 3 stade A à partir de 1,7 g du triflate obtenu à la préparation 15 et on obtient 1,1g de produit purifié attendu.
Rf=0,50 cyclohexane/acétate d'éthyle : 8/2
Spectre infrarouge (IR) Solvant : $CHCl_3$
1693cm$^{-1}$ carbonyle
1609, 1576, 1563, 1516, 1506cm$^{-1}$ aromatiques

Stade B : déprotection

3-chloro-4-(4-hydroxyphényl)-1-naphtalène-carboxaldéhyde (produit a) et 3-chloro 9-[4-hydroxy 3-(phénylméthyl) phényl] 1-naphtalène carboxaldéhyde (produit b).

On mélange pendant 10 minutes et sous atmosphère inerte, 150 mg de dérivé chloré du stade précédent et 3 ml d'acide trifluoroacétique. Après extraction à l'acétate d'éthyle et évaporation sous pression réduite, on purifie le produit brut par chromatographie en éluant avec le mélange cyclohexane-dichlorométhane/éther 70/20/10. On obtient 40 mg de produit attendu (a) que l'on utilise tel quel à l'étape suivante ainsi que 60 mg de l'analogue (b) déprotégé et réarrangé en position 3 du phényle (Rf=0,45 cyclohexane/acétate d'éthyle 70/30).
Rf = 0,40 cyclohexane/acétate d'éthyle : 7/3

Stade C : réduction

3-chloro-4-(4-hydroxyphényl)-1-naphtalène-méthanol

On procède de manière équivalente à l'exemple 2, à partir du produit (a) obtenu au stade précédent. On obtient 25 mg de produit pur attendu.
Rf=0,15 cyclohexane/acétate d'éthyle : 7/3
Microanalyse

| | | | | |
|---|---|---|---|---|
| % trouvé | C 71,6 | H 4,50 | Cl 12,5 | |
| % calculé | C 71,71 | H 4,6 | Cl 12,45 | O 11,24 |

**EXEMPLE 9 : 4-[4-hydroxy-3-(phénylméthyl)phényl]-3-chloro-1-naphtalèneméthanol**

On opère de manière équivalente à l'exemple 3, à partir de 60 mg du produit (b) obtenu à l'exemple 8 stade B et on obtient 40 mg de produit attendu.
Rf = 0,15 (cyclohexane/acétate d'éthyle 70/30
RMN ($CDCl_3$)
1,82 $CH_2O\underline{H}$
5,18 (s) $C\underline{H}_2OH$
4,07 (s) $C\underline{H}_2Ph$
4,82 (s) $Ph\text{-}O\underline{H}$
6,94 (dl) 1H; 7,10 (m) 2H; 7,15 à 7,47 5H; 7,42 1H; 7,53 2H; 7,69 (s) 1H; 8,09 (d) 1H : Aromatiques

**EXEMPLE 14 : 2,6-dichloro-alpha-éthynyl-4'-hydroxy-(1,1'-biphényl)-4-méthanol**

Stade A : couplage

2,6-dichloro-4'-[[(1,1-diméthyléthyl)diphénylsilyl]oxy]-(1,1'-biphényl)-4-carboxaldéhyde.

On opère de manière équivalente à l'exemple 3 à partir de 20,2 g de triflate obtenu à la préparation 12 dans 125 ml de dioxane et 22,4 g d'acide boronique obtenu à la préparation 3. Après purification par chromatographie en éluant avec le mélange cyclohexane/Chlorure de méthylène 92/8 puis 90/10 et enfin 80/20, on obtient 12,5 g d'un mélange constitué de 62 % de produit dichloré et 38 % de produit monochloré.
Rf=0,66 cyclohexane/acétate d'éthyle 7/3

Stade B : Addition du magnésien

2,6-dichloro-alpha-éthynyl-4'-[[(1,1-diméthyléthyl)diphénylsilyl]oxy]-(1,1'-biphényl)-4-méthanol

A une solution sous gaz inerte de 700 mg de produit silylé préparé au stade précédent dans 15 ml de THF, on additionne à 0-5 C, 1,5 ml de bromure d'éthynylmagnésium dans le THF et agite 2 heures 30 à cette température. Après neutralisation avec du chlorure d'ammonium saturé, on extrait à l'acétate d'éthyle, sèche, filtre et évapore sous pression réduite jusqu'à obtention de 720 mg de produit brut attendu.

Stade C : Déprotection du produit silylé

2,6-dichloro-alpha-éthynyl-4'-hydroxy-(1,1'-biphényl)-4-méthanol

On opère de manière équivalente à l'exemple 11 stade B et on obtient 90 mg de produit purifié.
Rf = 0,36 cyclohexane/acétate d'éthyle : 1/1
RMN ($CDCl_3$ 250MHz)

| | | |
|---|---|---|
| 2,75 | (d) | $CH(OH)\text{-}C\equiv C\text{-}\underline{H}$ |
| 5,47 | (sl, d après échange) | $C\underline{H}(OH)\text{-}C\equiv C\text{-}H$ |
| 2,32 | (m) et 4,92 (m) | 2H mobiles |

(suite)

| | | |
|---|---|---|
| 6,93 et 7,14 AA'BB' | | Ph-O |
| 7,59 | (s) | $H_3$; $H_5$ |

**EXEMPLE 15 : 2,6-dichloro-alpha-phényl-4'-hydroxy-(1,1'-biphényl)-4-méthanol**

On opère de manière équivalente à l'exemple 14 stades B et C par action du bromure de phényle magnésium sur 700 mg du produit silylé de l'exemple 14 stade A et on obtient 203 mg de produit attendu.
Rf = 0,5 cyclahexane/acétate d'éthyle : 1/1
RMN ($CDCl_3$ 250MHz)

| | | |
|---|---|---|
| 5,76 | (sl, d après échange) | C<u>H</u>(OH)-Ph |
| 6,17 | (d mobile) | |
| 6,81 et 7,01 AA'BB' | | Ph-O |
| 7,25 | (t) | $H_4$ du phényle |
| 7,35 | (t) | $H_3$, $H_5$ du phényle |
| 7,44 | (dl) | $H_2$, $H_6$ du phényle |
| 7,5 | (s) | $H_3$, $H_5$ |
| 9,61 | s mobile | Ph-O<u>H</u> |

**EXEMPLE 16 : 2,6-dichloro-alpha-éthyl-4'-hydroxy-(1,1'-biphényl)-4-méthanol**

On opère de manière équivalente à l'exemple 14 stades B et C par action du bromure d'éthyl magnesium sur 1,2 g du produit silylé de l'exemple 14 stade A, et on obtient 21 mg de produit attendu.
Rf = 0,47 cyclohexane/acétate d'éthyle : 1/1
RMN ($CDCl_3$ 250MHz)

| | | |
|---|---|---|
| 0,87 | t | $CH_2C\underline{H}_3$ |
| 1,63 | m | $C\underline{H}_2CH_3$ |
| 4,5 | m | C<u>H</u>OH |
| 5,39 | d | CHO<u>H</u> |
| 6,83 et 7,03 AA'BB' | | Ph-O |
| 7,45 | | $H_3$, $H_5$ |
| 9,62 | s | Ph-O<u>H</u> |

**EXEMPLE 18 : 2-bromo-4'-hydroxy-6-nitro-(1,1'-biphényl)-4-méthanol**

Stade A : couplage

2, 6-dinitro-4'-[[(1,1-diméthyléthyl)diméthylsilyl]oxy]-(1,1'-biphényl)-4-carboxylate de méthyle

On mélange sous atmosphère inerte 1,53 g de 4-chloro-3,5-dinitro benzoate de méthyle obtenu à l'exemple 30 stade A dans 15 ml de tétrahydrofuranne avec 1,96 g du produit de la préparation 5 et 2,8 g de cuivre puis on agite 24 heures à 120 C. Après filtration, on évapore sous pression réduite et purifie le produit brut par chromatographie en éluant avec le mélange cyclohexane/acétate d'éthyle 9/1 puis 1/1. On obtient ainsi 1,2g du produit attendu
(Rf=0,45 cyclohexane/acétate d'éthyle 7/3)
IR ($CHCl_3$)
C=O 1735$cm^{-1}$
Aromatiques + $NO_2$ : 1620, 1607, 1575, 1545, 1517 $cm^{-1}$

Stade B : réduction du groupe nitro

2-amino-4'-[[(1,1-diméthyléthyl)diméthylsilyl]oxy]-(1,1'-biphényl)-6-nitro-4-carboxylate de méthyle

On agite 16 heures sous atmosphère inerte, au reflux le mélange constitué de 1,15 g du produit obtenu au stade précédent, 4,9 ml de cyclohexène et 345 mg de $Pd(OH)_2$. Après filtration puis évaporation sous pression réduite, le produit brut est purifié par chromatographie en éluant avec le mélange cyclohexane/acétate d'éthyle 9/1. On obtient 670 mg d'une huile qui est cristallisée dans un mélange éther éthylique/hexane. On obtient ainsi 522 mg de produit attendu.
Rf=0,2 cyclohexane/acétate d'éthyle 85/15
IR ($CHCl_3$)

| | |
|---|---|
| $=C-NH_2$ | 3500 $cm^{-1}$ |
| C=O | 1726 $cm^{-1}$ |
| Aromatiques | 1621 $cm^{-1}$ |
| 1ere bande $NO_2$ | 1610 $cm^{-1}$ |
| $NH_2$ | 1635, 1516 $cm^{-1}$ |

Stade C : Substitution de $NH_2$ par Br

2-bromo-4'-[[((1,1-diméthyléthyl)diméthylsilyl]oxy]-(1,1'-biphényl)-6-nitro-4-carboxylate de méthyle

On ajoute sous atmosphère inerte 0,062 ml de terbutylnitrite à un mélange refroidi à -5°C, constitué du produit du stade précédent dans 1,3 ml de tribromométhyle. Après 15 minutes d'agitation à 100°C, on évapore sous pression réduite et le brut réactionnel est purifié par chromatographie en éluant avec un mélange cyclohexane/acétate d'éthyle 95/05. On obtient ainsi 81 mg du produit pur attendu.
Rf=0,33 cyclohexane/ acétate d'éthyle 9/1
IR ($CHCl_3$)
Absence de $=C-NH_2$

| | |
|---|---|
| C=O | 1730, 1436 $cm^{-1}$ |
| Aromatique | 1608, 1572 $cm^{-1}$ |
| 1ere bande $NO_2$ | 1537, 1516 $cm^{-1}$ |

Stade D : déprotection

2-bromo-4'-hydroxy-6-nitro-(1,1'-biphényl)-4-carboxylate de méthyle

On opère de manière équivalente à l'exemple 11 stade B à partir du produit obtenu au stade précédent. On obtient 279 mg de produit désilylé que l'on engage tel quel dans l'étape suivante.
Rf=0,38 cyclohexane/acétate d'éthyle : 9/1

Stade E : Réduction

2-bromo-4'-hydroxy-6-nitro-(1,1'-biphényl)-4-méthanol

On opère de manière équivalente à l'exemple 5 stade B à partir de 110 mg du produit préparé au stade précédent. On obtient ainsi 85 mg de produit attendu que l'on recristallise dans l'éther éthylique.
Rf=0,18 cyclohexane/acétate d'éthyle : 7/3
Microanalyse

| | | | | |
|---|---|---|---|---|
| % trouvé | C 48,2 | H 2,9 | N 4,1 | Br 24,8 |
| % calculé | C 48,2 | H 3,1 | N 4,3 | Br 24,7 |

**EXEMPLE 19 : 2-amino-6-bromo-4'-hydroxy-(1,1'-biphényl)-4-méthanol**

Stade A : réduction $NO_2$ + déprotection

2-amino-6-bromo-4'-hydroxy-(1,1'-biphényl)-4-carboxylate de méthyle

On procède de manière équivalente à l'exemple 18 stade B à partir de 10 g de produit obtenu au stade C de ce même exemple. On obtient 7,97 g de produit attendu (réduit et déprotégé) et 577 mg de l'analogue protégé (Rf=0,56 cyclohexane/acétate d'éthyle 7/3).
Rf=0,17 cyclohexane/acétate d'éthyle 7/3

Stade B : réduction de l'ester

2-amino-6-bromo-4'-hydroxy-(1,1'-biphényl)-4-méthanol

On opère de manière équivalente à l'exemple 5 stade B à partir de 550 mg produit obtenu au stade précédent. On obtient 250 mg de produit attendu.
Rf=0,18 cyclohexane/acétate d'éthyle 7/3
Microanalyse

| | | | | |
|---|---|---|---|---|
| % trouvé | C 52,8 | H 4,1 | N 4,5 | Br 26,9 |
| % calculé | C 53,1 | H 4,1 | N 4,8 | Br 27,2 |

IR (Nujol)
Absence de C=O
Absorption OH/NH
Région aromatique, $NH_2$ : 1606, 1586, 1550, 1540, 1516, 1496cm$^{-1}$

**EXEMPLE 20 : 2-bromo-4'-hydroxy-6-méthylthio-(1,1'-biphényl)-4-méthanol**

Stade A : Substitution de $NH_2$ par SMe

2-bromo-4'-[[(1,1-diméthyléthyl)diphénylsilyl]oxy]-6-méthylthio-(1,1'-biphényl)-4-carboxylate de méthyle

On agite 16 heures sous atmosphère inerte à température ambiante le mélange constitué de 400 mg du produit préparé à l'exemple 19 stade A (produit réduit et protégé) dans 1 ml de chloroforme, 0,17 ml de $(CH_3S)_2$ et 0,16 ml de terbutylnitrite. Après hydrolyse du milieu réactionnel, extraction et concentration sous pression réduite, le produit brut est purifié par chromatographie en éluant avec le mélange cyclohexane/acétate d'éthyle 998/02. On obtient ainsi 245 mg de produit attendu.
Rf=0,47 cyclohexane/acétate d'éthyle 1/1
RMN ($CDCl_3$ 200MHz)

| | | |
|---|---|---|
| 0,19 | s | Si(C$\underline{H}_3$)$_2$ |
| 0,9 | s | Si-C($CH_3$)$_3$ |
| 2, 3 | s | S-C$\underline{H}_3$ |
| 6,85 et 7,0 | AA'BB' | Ph-O |
| 7,68 d et 8,0 d | | $H_3$, $H_5$ |

Stade B : Réduction + Déprotection

2-bromo-4'-hydroxy-6-méthylthio-(1,1'-biphényl)-4-méthanol

On opère de manière équivalente à l'exemple 18 stades D et E à partir de 200 mg du produit obtenu au stade précédent. On obtient ainsi 10,9 mg de produit pur attendu.
Rf=0,2 cyclohexane/acétate d'éthyle 7/3
RMN (DMSO, 250MHz)

| | | |
|---|---|---|
| 2,31 | s | S-C<u>H</u>$_3$ |
| 4,53 | s | C<u>H</u>$_2$OH |
| 5,34 (t, mobile) | | CH$_2$O<u>H</u> |
| 6,81 et 6,93 AA'BB' | | Ph-O |
| 7,17 (sl) et 7,39 (sl) | | $H_3$, $H_5$ |
| 9,54 (s,l) | | Ph-OH |

**EXEMPLE 21 : 2-bromo-4'-hydroxy-6-(1H-pyrrol-1-yl)-(1,1'-biphényl)-4-méthanol**

Stade A : Formation du pyrrole à partir de l'amine

2-bromo-4'-[[(1,1-diméthyléthyl)diphénylsilyl]oxy]-6-(1H-pyrrol-1-yl)-(1,1'-biphényl)-4-carboxylate de méthyle

On mélange sous atmosphère inerte 700 mg du produit préparé à l'exemple 19 (produit réduit et protégé) dans 17 ml d'acide acétique et 23 ml de 2,6-diméthoxy tétrahydrofuranne et on porte 16 heures au reflux. Après hydrolyse du milieu réactionnel, extraction à l'acétate d'éthyle et évaporation sous pression réduite, on obtient 813 mg de produit brut attendu que l'on utilise tel quel pour l'étape suivante.
Rf= 0,8 cyclohexane/acétate d'éthyle
RMN ($CDCl_3$, 200MHz)

| | | |
|---|---|---|
| 0,12 | s | Si(C<u>H</u>$_3$)$_2$ |
| 0,85 | s | Si-C(C<u>H</u>$_3$)$_3$ |
| 3,85 | s | CO$_2$C<u>H</u>$_3$ |
| 5,95 | m | $H_3$, $H_4$ du pyrrol |
| 6,56 | m | $H_2$, $H_5$ du pyrrol |
| 6,66 et 6,87 | | AA'BB' Ph-O |
| 7,31 et 7,67 | | $H_3$, $H_5$ |

Stade B : Réduction et déprotection

2-bromo-4'-hydroxy-6-(1H-pyrrol-1-yl)-(1,1'-biphényl)-4-méthanol

On opère de manière équivalente à l'exemple 18 stades D et E à partir de 750 mg de produit obtenu au stade précédent. On obtient ainsi 212 mg de produit pur attendu.
Rf=0,29 cyclohexane/acétate d'éthyle 7/3
RMN (DMSO, 250MHz)

| | | |
|---|---|---|
| 5,42 | t, mobile | CH$_2$O<u>H</u> |
| 4,57 | d, s après échange | C<u>H</u>$_2$OH |
| 5,95 | m | $H_3$, $H_4$ du pyrrol |
| 6,56 | m | $H_2$, $H_5$ du pyrrol |
| 6,66 et 6,87 AA'BB' | | Ph-OH |
| 7,31 et 7,67 d | | $H_3$, $H_5$ |
| 9,44 | s mobile | Ph-O<u>H</u> |

**EXEMPLE 22 : 2,6-diméthoxy-4'-hydroxy-(l,l'-biphéayl)-4-méthanol**

Stade A : Couplage

2,6-diméthoxy-4'-benzyloxy-(1,1'-biphényl)-4-carboxaldéhyde

A 5,07 g de 4-bromo-3,5-diméthoxy-benzaldéhyde (Finorga) dans 50 ml de dioxane, on ajoute, sous gaz inerte, 19 g d'acide boronique obtenu à la préparation 2, 7,15 g de tri-potassium phosphate 1-hydrate et 1,2 g de Tétrakis

(triphénylphosphine)-palladium(0). On porte au reflux pendant 15 heures, verse le mélange réactionnel dans de l'eau glacée et extrait la phase aqueuse avec de l'éther puis de l'acétate d'éthyle. Les phases organiques sont séchées et évaporées sous pression réduite. Le produit brut ainsi récupéré est chromatographié sur silice en éluant avec un mélange dichlorométhane/pentane 70/30. On recueille ainsi 2,84 g de produit attendu Rf = 0,5 dichlorométhane
I.R. $CHCl_3$

| | |
|---|---|
| C=O | 1692 $cm^{-1}$ |
| Aromatiques | 1610, 1582, 1572, 1520, 1499 $cm^{-1}$ |

Stade B : Débenzylation et réduction

2,6-diméthoxy-4'-hydroxy-(1,1'-biphényl)-4-méthanol

On mélange à température ambiante 502,3 mg de produit obtenu au stade précédent dans 13 ml d'acétate d'éthyle avec 161 mg de palladium sur charbon à 9,5 %. On place le milieu réactionnel sous atmosphère d'hydrogène et agite pendant 3 heures. Après filtration, le filtrat est évaporé sous pression réduite et le résidu brut est chromatographié sur silice en éluant avec d'abord du dichlorométhane puis un mélange dichlorométhane/méthanol 95/5. On recueille ainsi 134 mg de produit attendu ainsi que 110 mg du produit de l'exemple 23 ($R_5$=Me).
Rf = 0,41 dichlorométhane/méthanol 9/1
F = 184 C
I.R. Nujol
Absence de C=O

| | |
|---|---|
| Région OH/NH max | 3500 $cm^{-1}$ |
| | max 3260 $cm^{-1}$ |
| Aromatiques | 1610, 1593, 1579, 1523, 1492 $cm^{-1}$ |

**EXEMPLE 24 : 2,6-dichloro-4'-hydroxy-(1,1'-biphényl)-4-méthanol**

Stade A : Protection

2-(3,5-dichlorophényl)-1,3-dioxolane

On mélange 4,66 g de 3,5-dichlorobenzaldéhyde avec 0,23 g d'acide paratoluènesulfonique, 3 ml d'éthylène glycol et 40 ml de toluène. On chauffe au reflux pendant 2 heures, verse le mélange réactionnel sur une solution saturée en bicarbonate de sodium et décante la phase aqueuse. La phase organique est séchée et évaporée sous pression réduite. On isole 5,756 g du produit attendu sous la forme d'huile.
Rf = 0,45 cyclohexane/acétate d'éthyle 9/1.
**I.R.** $CHCl_3$
Absence de C=O

Aromatiques 1592, 1575 $cm^{-1}$

Cétal

Stade B : Orthométallation et iodation

2-(3,5-dichloro-4-iodo phényl)-1,3-dioxolane

On refroidit à -78°C sous atmosphère inerte, une solution de 5,631 g de produit obtenu au stade précédent dans 45 ml de tétrahydrofuranne, ajoute en 1 heure, goutte à goutte, 21,4 ml d'une solution de n-butyllithium, agite à -78°C pendant 30 minutes, puis ajoute en 1 heure, goutte à goutte une solution de 6,94 g de N-iodosuccinimide dans 40 ml de tétrahydrofuranne. On agite pendant 1 heure à -78°C, verse le mélange réactionnel dans une solution saturée en chlorure d'ammonium et extrait avec du chlorure de méthylène. La phase organique est séchée et évaporée sous pression réduite. On isole 10,11 g du produit attendu sous la forme d'huile.
Rf = 0,22 cyclohexane/acétate d'éthyle 95/5.

Stade C : Couplage

2-[2,6-dichloro-4'-phénylméthoxy-(1,1'-biphényl)-4-yl]-1,3-dioxolane

On mélange 10,11 g du produit obtenu au stade précédent avec 60 ml de toluène, 10,69 g d'acide boronique (préparation 2), 29,3 ml d'une solution (2M) de carbonate de sodium, 15 ml d'éthanol et 1,69 g de Tétrakis(triphénylphosphine)-palladium (0). On agite au reflux du toluène sous atmosphère inerte pendant 15 heures et on verse le mélange réactionnel dans une solution saturée en bicarbonate de sodium et extrait avec de l'éther. La phase organique est lavée avec une solution saturée en chlorure d'ammonium, puis avec une solution de chlorure de sodium, séchée et évaporée sous pression réduite. On isole 14,43 g du produit attendu sous la forme d'huile.
Rf = 0,2 cyclohexane/acétate d'éthyle 90/10.
I.R. $CHCl_3$
aromatiques 1611, 1580, 1555, 1520, 1498 $cm^{-1}$

Stade D : Déprotection (Hydrolyse du cétal)

2,6-dichloro-4'-phénylméthoxy-(1,1'-biphényl)-4-carboxaldéhyde

On mélange 14,31 g du produit obtenu au stade précédent avec 70 ml de tétrahydrofuranne et 36 ml d'acide chlorhydrique 1N. On porte au reflux du tétrahydrofuranne pendant 15 heures et traite le mélange réactionnel avec une solution saturée en bicarbonate de sodium et extrait la phase aqueuse avec du dichlorométhane. La phase organique est séchée et évaporée sous pression réduite. 13,24 g de produit brut sous forme d'une huile sont chromatographiés sur silice en éluant avec le mélange cyclohexane/acétate d'éthyle 99,5/0,5. On recueille ainsi 2,352 g de produit attendu.
Rf = 0,4 cyclohexane/acétate d'éthyle 90/10
I.R. CHCl3
aromatiques 1609, 1590, 1577, 1548, 1516 $cm^{-1}$
C=O 1707 $cm^{-1}$

Stade E : Débenzylation

2,6-dichloro-4'-hydroxy-(1,1'-biphényl)-4-méthanol

On mélange à température ambiante 101,4 mg de produit obtenu au stade précédent dans 5 ml d'acétate d'éthyle avec 31 mg de palladium sur charbon à 9,5 %. On place le milieu réactionnel sous atmosphère d'hydrogène et agite pendant 4 heures à 20 C. Après filtration, le filtrat est évaporé sous pression réduite puis on purifie par chromatographie sur silice en éluant avec un mélange cyclohexane/acétate d'éthyle 9/1. On recueille ainsi 61 mg de produit attendu.
Rf = 0,21 cyclohexane/acétate d'éthyle 7/3
F= 161 C
I.R. $CHCl_3$
-OH 3599 $cm^{-1}$
Aromatiques 1613,1603, 1593, 1546, 1520, 1501 $cm^{-1}$

**EXEMPLE 26 : 4'-hydroxy-2,3,5,6-tétrachloro-(1,1'-biphényl)-4-méthanol**

Stade A : Couplage

4'-phénylméthoxy-2,3,5,6-tétrachloro-(1,1'-biphényl)

On mélange 1 g de 3-iodo-1,2,4,5-tétrachlorobenzène, 790 mg d'acide boronique obtenu à la préparation 2, 80 mg de palladium tétrakis et 1 g de phosphate de potassium dans 100 ml de dioxane. On porte au reflux pendant 5 heures, ajoute à nouveau 80 mg de palladium tétrakis et porte au reflux pendant 24 heures. Le mélange est directement chromatographié sur silice en éluant avec un mélange cyclohexane/acétate d'éthyle 99/1. On recueille ainsi 689 mg de produit brut que l'on recristallise dans un mélange éther-pentane. On obtient le produit attendu avec un rendement de 60 %.
Rf = 0,6 cyclohexane/acétate d'éthyle 95/5
I.R. $CHCl_3$
Aromatiques : 1601, 1590, 1500 $cm^{-1}$

Stade B : Formylation

4'-phénylméthoxy-2,3,5,6-tétrachloro-(1,1'-biphényl)-4-carboxaldéhyde

On refroidit à -78°C, 350 mg du produit préparé au stade précédent dans 5 ml de tétrahydrofuranne, ajoute goutte à goutte 0,69 ml d'une solution de n-butyllithium 1,45M dans l'hexane puis après 2 heures d'agitation à -78°C 90 µl de diméthylformamide dans 0,4 ml de tétrahydrofuranne. On agite 16 heures à -65°C, coule le milieu réactionnel dans 13,6 g de glace et 2,7 ml d'acide chlorhydrique concentré et extrait la phase aqueuse avec de l'acétate d'éthyle. Après séchage et concentration on obtient 320 mg d'un mélange composé par le produit attendu et le produit de départ que l'on utilise directement au stade suivant.
Rf = 0,47 cyclohexane/acétate d'éthyle 95/5
RMN ($CDCl_3$) 200MHz

| | | |
|---|---|---|
| 10,35 | (s) | C<u>H</u>O |
| 7 | (m) | H aromatiques Ph-0 |
| 7,4 | (m) | H aromatiques benzyle |
| 5,05 | (s) | Ph-$C\underline{H}_2$-O |

Stade C : Réduction

4'-phénylméthoxy-2,3,5,6-tétrachloro-(1,1'-biphényl)-4-méthanol

On mélange 320 mg du produit préparé au stade précédent avec 16 ml de méthanol, 34 mg de borohydrure de sodium et 0,5 ml de dichlorométhane et agite 15 minutes à température ambiante. On verse ensuite le mélange réactionnel dans une solution saturée en chlorure d'ammonium, extrait la phase aqueuse avec de l'acétate d'éthyle, sèche et évapore sous pression réduite. Le résidu est chromatographié sur silice en éluant avec le mélange acétate d'éthyle/cyclohexane 10/90. On obtient ainsi 130 mg du produit attendu.
Rf = 0,56 cyclohexane/acétate d'éthyle 95/5.

Stade D: Déprotection

4'-hydroxy-2,3,5,6-tétrachloro-(1,1'-biphényl)-4-méthanol

On mélange à température ambiante 130 mg de produit obtenu au stade précédent dans 5,5 ml d'acétate d'éthyle avec 34 mg de palladium sur charbon à 9,5 %. On place le milieu réactionnel sous atmosphère d'hydrogène et agite pendant 6 heures à 20°C. Après filtration, le filtrat est évaporé sous pression réduite puis on purifie par chromatographie sur silice en éluant avec un mélange cyclohexane/acétate d'éthyle 9/1. On recueille ainsi 116 mg de produit brut que l'on recristallise dans le dichlorométhane. On obtient 47 mg du produit attendu.
Rf = 0,14 cyclohexane/acétate d'éthyle 8/2
RMN ($CD_3COCD_3$) 250MHz

| | | |
|---|---|---|
| 2,23 | (m) | $CH_2O\underline{H}$ |
| 4,97 large | | Ph-O<u>H</u> |
| 5,13 | (s) | $C\underline{H}_2OH$ |
| 7,10, 6,96 | (dd) | H aromatiques Ph-OH |

**EXEMPLE 27 : 2,6-difluoro-4'-hydroxy-(1,1'-biphényl)-4-méthanol**

Stade A : Protection

1,3-difluoro 5-[(tétrahydropyrannyloxy)méthyl]- benzène

On mélange sous atmosphère inerte 1,286 g de 3,5-difluorophényl méthanol avec 1,3 ml de 3,4-dihydropyranne, 155 mg d'acide paratoluènesulfonique et 20 ml de dioxane et on agite à 20°C pendant 2 heures 30 minutes. On verse le mélange réactionnel dans une solution saturée en bicarbonate de sodium, évapore le dioxane sous pression réduite et extrait la phase aqueuse avec de l'acétate d'éthyle. La phase organique est séchée puis évaporée sous pression

réduite. On recueille 1,81 g de produit attendu.
Rf = 0,42 cyclohexane/acétate d'éthyle 9/1
I.R. $CHCl_3$
Absence d'OH

Aromatiques 1632, 1602 $cm^{-1}$

Stade B : Chloration, couplage et déprotection de l'alcool 2,6-difluoro-4'-phénylméthoxy-(1,1'-biphényl)-4-méthanol

On refroidit à -78°C une solution de 1,04 g du produit obtenu au stade précédent dans 20 ml de tétrahydrofuranne et on ajoute goutte à goutte sous atmosphère inerte 3,7 ml d'une solution de n-Butyllithium (1,5M dans l'hexane) puis, après 15 minutes d'agitation à -78°C, on ajoute 5,5 ml d'une solution de chlorure de Zinc (1,0M dans le tétrahydrofuranne). Après 30 minutes d'agitation à -78°C, on laisse remonter la température à 20°C et on ajoute 1,44 g du produit obtenu au stade A de la préparation 2 et 265 mg de Tétrakis (triphénylphosphine)-palladium (0). On porte au reflux pendant 5 heures, verse le mélange réactionnel dans une solution saturée en chlorure d'ammonium et extrait la phase aqueuse avec du dichlorométhane. La phase organique est séchée et concentrée sous pression réduite. Le produit brut sous forme d'huile est utilisé tel quel au stade suivant de déprotection de l'alcool.

L'huile brute est dissoute dans 30 ml de méthanol et on rajoute 2,3 ml d'acide chlorhydrique 2N. On agite pendant 4 heures à 20°C et verse dans une solution saturée en bicarbonate de sodium. Après concentration du méthanol sous pression réduite, la phase aqueuse est extraite avec du dichlorométhane, la phase organique est séchée et concentrée sous pression réduite. On obtient ainsi 1,851 g du produit attendu.
Rf = 0,32 cyclohexane/acétate d'éthyle 7/3
I.R. Nujol
Absorption complexe région OH/NH
Aromatiques 1640, 1612, 1582,1569, 1528, 1492 $cm^{-1}$

Stade C : Débenzylation

2,6-difluoro-4'-hydroxy-(1,1'-biphényl)-4-méthanol

On mélange à température ambiante 32 mg de produit obtenu au stade précédent dans 5 ml d'acétate d'éthyle avec 31 mg de palladium sur charbon à 9,5 %. On place le milieu réactionnel sous atmosphère d'hydrogène et agite pendant 28 heures à 20°C. Après filtration, le filtrat est évaporé sous pression réduite. On recueille ainsi 116 mg de produit brut que l'on recristallise dans le dichlorométhane. On obtient 18 mg du produit attendu.
Rf = 0,18 cyclohexane/acétate d'éthyle 7/3
I.R. Nujol
Absorption complexe région OH/NH
Aromatiques 1636, 1615, 1598,1573, 1530 $cm^{-1}$

**EXEMPLE 28 : 4'-hydroxy-2-trifluorométhyle- (1,1'-biphényl) -4-méthanol**

Stade A : Couplage

4'-benzyloxy-2-trifluorométhyle-(1,1'-biphényl)-4-carboxaldéhyde

En opérant comme à l'exemple 22 Stade A, en partant de 0,911 g de (3-trifluorométhyle-4-bromo-benzaldéhyde) et après purification par chromatographie sur colonne de silice en éluant avec un mélange cyclohexane/acétate d'éthyle 95/5, on obtient 1,093 g de produit attendu.
Rf = 0,33 cyclohexane/acétate d'éthyle (8/2)
I.R. ($CHCl_3$)
CHO 2736, 1706 $cm^{-1}$
Ar 1611 (F), 1580, 1565 (ep.) et 1520 $cm^{-1}$

Stade B : Déprotection et réduction

4'-hydroxy-2-trifluorométhyle-(1,1'-biphényl)-4-méthanol

On mélange à température ambiante 308 mg de produit obtenu au stade précédent dans 15 ml d'acétate d'éthyle avec 290 mg de palladium sur charbon à 9,5 %. On place le milieu réactionnel sous atmosphère d'hydrogène et agite pendant 12 heures à 20°C. Après filtration, le filtrat est évaporé sous pression réduite. On obtient 215,7 mg d'un mélange de produit débenzylé et de son analogue réduit que l'on engage directement dans la réaction suivante.

On mélange 215,7 mg du mélange préparé au stade précédent avec 3 ml de méthanol, 48 mg de borohydrure de sodium à 95 % et agite 3 heures à température ambiante. Après concentration sous pression réduite, on reprend mélange réactionnel avec du dichlorométhane et lave avec une solution saturée en chlorure d'ammonium, sèche et évapore sous pression réduite. Le brut réactionnel est chromatographié sur silice en éluant avec le mélange acétate d'éthyle/cyclohexane 2/8. On obtient ainsi 99 g du produit attendu.
Rf = 0,20 cyclohexane/acétate d'éthyle 7/3)
I.R. (Nujo1)
Absence de C=O
Absorption générale région OH/NH
1615, 1600, 1520 (ép.) et 1492 $cm^{-1}$ Ar

**<u>EXEMPLE 29 :</u> 4'-méthyl-2'-trifluorométhyl-(1,1'-biphényl)-4-ol**

On opère comme à l'exemple 3 à partir de 1,004 g de biphényle obtenu au stade A de l'exemple précédent et on obtient 216 mg du produit attendu ainsi que 373 mg du produit de l'exemple 28 ($R_5$=$CH_2OH$)
Rf = 0,49 cyclohexane/acétate d'éthyle 7/3
RMN $CDCl_3$/250MHz

| | | | |
|---|---|---|---|
| 2,44 | (s) | | C<u>H</u>$_3$ |
| 7,19 | (masqué) | | H'$_6$ |
| 7,34 | (dm) | | H'$_5$ |
| 7,53 | (m) | | H'$_3$ |
| 6,85 et 7,20 | | AA'BB' | Ph-O- |
| 4,74 | (s) | | 1H mobile |

**<u>EXEMPLE 30</u>: 2,6-dinitro-4'-hydroxy-(1,1'-biphényl)-4-méthanol**

Stade A : Estérification

4-chloro-3,5-dinitro-benzoate de méthyle

On ajoute à 0°C, sous atmosphère inerte et goutte à goutte 2,96 ml de $SOCl_2$ à 10 g d'acide 3,5-dinitro-4-chloro-benzoique (JANSSEN) dans 150 ml de méthanol et on agite au reflux du méthanol pendant 2 heures puis 15 heures à température ambiante. On verse le milieu réactionnel dans de la glace et observe la précipitation du produit. Après séchage, on obtient 10,3 g de produit attendu.
Rf = 0,45 cyclohexane/acétate d'éthyle 7/3
I.R. $CHCl_3$

| | |
|---|---|
| C=O | 1738 $cm^{-1}$ |
| Aromatiques + NO2 | 1614, 1552 $cm^{-1}$ |

Stade B : Couplage

2,6-dinitro-4'-phénylméthoxy-(1,1'-biphényl)-4-carboxylate de méthyle

On mélange sous atmosphère inerte 10 g du produit du stade précédent dans 100 ml de diméthylformamide avec 8,4 g du produit de la préparation 6 et 15,12 g de cuivre puis on agite en amenant la température de 20 à 120°C en 2 heures et en maintenant 30 minutes à 120°C. Après filtration du brut réactionnel, on verse dans un mélange glace

plus eau et extrait avec du dichlorométhane. La phase organique est séchée, concentrée sous pression réduite et chromatographiée sur silice en éluant avec un mélange cyclohexane/acétate d'éthyle 8/2. On recueille ainsi 9,1 g de produit attendu.
Rf = 0,46 cyclohexane/acétate d'éthyle 7/3
RMN $CDCl_3$/200MHz

| | | |
|---|---|---|
| 4 | (s) | COOMe |
| 7-'7, 15 | | Ph-O |
| 7, 35 | | Ph-$CH_2$-O |
| 8,5 | | 2H ortho du COOMe |
| 5 | | Ph-$CH_2$-O |

Stade C : Déprotection

2,6-dinitro 4'-hydroxy (1,1'-biphényl) 4-carboxylate de méthyle

On ajoute 11 ml d'acide trifluoroacétique à 1,3 g de produit obtenu au stade précédent et on agite au reflux de l'acide acétique pendant 1 heure 30. On verse le milieu réactionnel sur un mélange glace plus eau et extrait avec du dichlorométhane. La phase organique est séchée, concentrée sous pression réduite et chromatographiée sur silice en éluant avec un mélange cyclohexane/acétate d'éthyle 7/3. On recueille ainsi 330 mg de produit attendu.
Rf = 0,42 cyclohexane/acétate d'éthyle 7/3)
RMN $CDCl_3$/200MHz

| | |
|---|---|
| 3,98 (s) | COOMe |
| 6,8-7,1 | 4H aromatiques |
| 8,49 | 2H ortho du COOMe |

Stade D : Réduction

2,6-dinitro-4'-hydroxy-(1,1'-biphényl)-4-méthanol

On procède de manière équivalente à l'exemple 5 stade B à partir de 330 mg du produit préparé au stade précédent.

Après purification par chromatographie sur silice en éluant avec un mélange cyclohexane/acétate d'éthyle 7/3 On recueille 71,8 mg de produit attendu.
Rf = 0,45 cyclohexane/acétate d'éthyle 7/3
F = 156-158°C
RMN DMSO/250MHz

| | | |
|---|---|---|
| 4,69 | (d) | Ph-$CH_2$OH |
| 5,73 | (t) | Ph-$CH_2$OH |
| 6,81 et 7,05 | AA'BB' | Ph-O- |
| 8,15 | (s) | $H_3$, $H_5$ |
| 9,82 | (s) 1H mobile | |

**EXEMPLE 31: 2 -amino-4'-hydroxy-6-nitro-(1,1'-biphényl)-4-méthanol**

On mélange sous atmosphère inerte 300 mg de produit préparé à l'exemple 30 dans 6 ml de tétrahydrofuranne avec 0,64 ml de cyclohexène et 30 mg de Pd(OH)2 et on agite 3 heures à température ambiante puis 16 heures au reflux de tétrahydrofuranne. Après filtration on évapore le tétrahydrofuranne, reprend avec de l'acétate d'éthyle et extrait la phase organique avec de l'acide chlorhydrique 2N. La phase acide est ramenée à pH basique avec de la soude 2N, puis est extraite avec de l'acétate d'éthyle. La phase organique est séchée, concentrée sous pression réduite. Le produit brut ainsi obtenu est recristallisé dans le chloroforme. On obtient ainsi 26 mg de produit attendu.
Rf = 0,11 dichlorométhane/méthanol 95/05)
F=158°C

RMN DMSO/250MHz

| | | |
|---|---|---|
| 4,44 | (sl) | Ph-C$\underline{H}_2$OH |
| 5,02 | (sl) | $NH_2$ |
| 5,30 | (sl) | H mobile |
| 5,73 | (t) | Ph-$CH_2$O$\underline{H}$ |
| 6,83 | (d) et 6,98 (d) | |
| 6,91 | (sl) et 6,93 (sl) | $H_3$, $H_5$ $CH_2OH$ |
| 9,57 | (s) 1H mobile | |

**EXEMPLE 32 : 2-bromo-4'-hydroxy-6-iodo-(1,1'-biphényl)-4-méthanol**

Stade A : iodation du biphényl aminé

2-bromo-4'-[[(1,1-diméthyléthyl)diméthylsilyl]oxy]-6-iodo-(1,1'-biphényl)-4-carboxylate de méthyle

On ajoute 4 g d'iode à une solution de 2,69 g de produit préparé au stade A de l'exemple 19, dans 50 ml de chloroforme, puis, goutte à goutte, 1,05 ml de terbutylnitrite et laisse sous agitation au reflux pendant 1 heure 30. Après neutralisation de l'excès d'iode par une solution de thiosulfate de sodium, on extrait au chloroforme, sèche et évapore sous pression réduite. Le produit brut est purifié par chromatographie sur colonne en éluant avec un mélange cyclohexane/acétate d'éthyle 99/1. On obtient 3,075 g de produit attendu.

| | | |
|---|---|---|
| Rf=0,70 | cyclohexane/acétate d'éthyle 80/20 | |
| RMN $CDCl_3$ | 250MHz | |
| 0,25 | s | Si(C$\underline{H}_3$)$_2$ |
| 1,01 | s | Si-C(C$\underline{H}_3$)$_3$ |
| 3,94 | s | $CO_2$C$\underline{H}_3$ |
| 6,96 AA'BB' | | Ph-O |
| 8,29 d et 8,52 d $H_3$, $H_5$ | | |

Stade B : Réduction

2-bromo-4'-[[(1,1-diméthyléthyl)diméthylsilyl]oxy]-6-iodo-(1,1'-biphényl)-4-méthanol

On ajoute sous atmosphère inerte à -70°C, à une solution de 160 mg de l'ester préparé au stade précédent dans 2 ml de toluène, 2 équivalents d'une solution extemporanée de diiso-butylaluminium/nbutyl lithium/toluène/n-hexane, agite 3 heures à -70°C et ajoute 19,4 mg de borohydrure de sodium dans 0,5 ml de méthanol. Après 45 minutes d'agitation en dehors du bain froid, le milieu est versé dans de l'eau glacée puis on extrait à l'acétate d'éthyle. Après séchage, puis évaporation sous pression réduite, le produit brut obtenu est purifié par chromatographie en éluant avec un mélange cyclohexane/acétate d'éthyle 98/2 puis 95/5. On obtient 141 mg de produit attendu que l'on engage tel quel dans le stade suivant.
Rf=0,48 cycohexane/acétate d'éthyle 7/3

Stade C : déprotection du phénol

2-bromo-4'-hydroxy-6-iodo-(1,1'-biphényl)-4-méthanol

On opère de la même manière qu'à l'exemple 11 stade B à partir de 141 mg du produit obtenu au stade précédent. On obtient 81 mg de produit attendu.
Rf = 0,26 cyclohexane/acétate d'éthyle 7/3
RMN

| | | |
|---|---|---|
| 4,49 | d | C$\underline{H}_2$OH |
| 5,40 | t | $CH_2$O$\underline{H}$ |
| 7,65 et 7,88 | d | $H_3$, $H_5$ |

(suite)

| | | |
|---|---|---|
| 6,8 à 6,92 | AA'BB' | Ph-OH |
| 9,58 | | Ph-OH |

**EXEMPLE 33 : 2-bromo-4'-hydroxy-6-[3-(diméthylamino)-1-propynyl]-(1,1'-biphényl)-4-méthanol**

Stade A : Couplage du diaryle bromé avec la propargylamine 2-bromo-6-(3-diméthylamino-1-propynyl)-4'-[[(1,1-diméthyléthyl)diméthylsilyl]oxy]-(1,1'-biphényl)-4-carboxylate de méthyle

A une solution sous atmosphère inerte de 226,7 mg de produit obtenu au stade A de l'exemple 32 dans 4 ml de DMF, on ajoute 2 ml de diéthylamine, 4 mg de iodure de cuivre, 24 mg de tétrakispalladium et 0,07 ml de propargylamine, puis on porte au reflux pendant 30 minutes. Après avoir versé le milieu réactionnel dans de l'eau glacée, on extrait à l'acétate d'éthyle, sèche, évapore sous pression réduite et purifie par chromatographie en éluant avec le mélange cyclohexane/acétate d'éthyle 60/40. On obtient 82,3 mg de produit attendu.
Rf=0,49 acétate d'éthyle 100 %

RMN $CDCl_3$ 250MHz

| | | |
|---|---|---|
| 0,23 | s | $Si(C\underline{H}_3)_2$ |
| 1,00 | s | $Si-C(C\underline{H}_3)_3$ |
| 2,08 | s | $N(C\underline{H}_3)_2$ |
| 3,29 | s | $\equiv C-C\underline{H}_2-N$ |
| 3,95 | s | $CO_2C\underline{H}_3$ |
| 6,90 et 7,17 | AA'BB' | Ph-O |
| 8,12 d et 8,24 d | | $H_3$, $H_5$ |

Stade B : réduction et déprotection

2-bromo-4'-hydroxy-6-[3-(diméthylamino)-1-propynyl]-(1,1'-biphényl)-4-méthanol

On opère de manière équivalente à l'exemple 32 stades B et C, à partir de 367 mg de produit obtenu au stade précédent. On obtient 35 mg du produit attendu A ainsi que 44 mg de produit B ($R_5$=CH(OH)nBu, Rf=0,36 dichlorométhane/méthanol: 9/1).

Produit A :

Rf=0,25 dichlorométhane/méthanol 9/1

RMN DMSO 250MHz

| | | |
|---|---|---|
| 1,98 | s | $N(C\underline{H}_3)_2$ |
| 3,29 | s | $\equiv C-C\underline{H}_2-N$ |
| 4,50 | d | $C\underline{H}_2OH$ |
| 5,38 | t, mobile | $CH_2O\underline{H}$ |
| 6,80 et 7,05 | AA'BB' | Ph-O |
| 7,44 d et 7,62 d | | $H_3$, $H_5$ |
| 9,51 H mobile | | Ph-OH |

**Produit B :** 2-bromo-alpha-butyl-4'-hydroxy-6-[3-(diméthylamino)-1-propynyl]-(1,1'biphényl)-méthanol

RMN $CDCl_3$ 250MHz

| | | |
|---|---|---|
| 0,92 | t | $CH_2-CH_2-CH_2-C\underline{H}_3$ |
| 1,36 | m (4H) | $CH_2-C\underline{H}_2-C\underline{H}_2-CH_3$ |
| 1,76 | m | $C\underline{H}_2-CH_2-CH_2-CH_3$ |
| 2,09 | s | $N(C\underline{H}_3)_2$ |

(suite)

| | | | |
|---|---|---|---|
| 3,30 | s | | ≡C-C$\underline{H}_2$-N |
| 4,63 | dd | | C$\underline{H}$(nBu)OH |
| 5,38 | t, mobile | | $CH_2O\underline{H}$ |
| 6,82 et 7,14 | | AA'BB' | Ph-OH |
| 7,44 d et 7,61 d | | | $H_3$, $H_5$ |

**<u>EXEMPLE 34</u> : 2-(3-diméthylamino-1-propynyl)-4'-hydroxy-6-nitro-(1,1'-biphényl)-4-méthanol**

On opère de manière équivalente à l'exemple 33 stades A et B à partir de 474,5 mg de produit préparé à l'exemple 18 stade C. On obtient 33 mg du produit attendu recristallisé dans l'éther éthylique.
Rf=0,14 $CH_2Cl_2$/MeOH 90/10
RMN (DMSO, 250MHz)

| | | | |
|---|---|---|---|
| 1,99 | s | | N(C$\underline{H}_3$)$_2$. |
| 3,29 | s | | ≡C-C$\underline{H}_2$-N |
| 4,59 | d | | C$\underline{H}_2$OH |
| 5,52 | t | | $CH_2O\underline{H}$ |
| 6,79 et 7,08 | d AA'BB4 | | Ph-O |
| 7,71 et 7,71 | d | | $H_3$, $H_5$ |
| 9,12 | sl | H mobile | Ph-O$\underline{H}$ |

**<u>EXEMPLE 35</u> : 4,4"-dihydroxy-(1,1':2',1"-terphényl)-5'-méthanol**

<u>Stade A :</u> déméthylation

4,4"-dihydroxy-(1,1':2',1"-terphényl)-5'-carboxylate de méthyle

On refroidit à -30°C, sous atmosphère inerte 2,7 g de 4,4"-diméthoxy-(1,1':2',1"-terphényl)-5'-carboxylate de méthyle (produit préparé selon J. Med. Chem. (1989) <u>32</u> 1814-1820) dans 30 ml de dichlorométhane, ajoute 2,7 ml de tribromoborane et agite à -30°C pendant 16 heures. On dilue dans une solution saturée en acétate de sodium, extrait la phase aqueuse avec de l'acétate d'éthyle, sèche la phase organique puis l'évapore. Le brut réactionnel est chromatographié sur silice en éluant avec un mélange cyclohexane/acétate d'éthyle 70/30. On obtient ainsi 814 mg de produit attendu.
Rf = 0,31 cyclohexane/acétate d'éthyle 1/1

<u>Stade B</u> : Acétylation et réduction

4,4"-dihydroxy-(1,1':2',1"-terphényl)-5'-méthanol

On refroidit sous atmosphère inerte à 0°C, 260 mg du produit obtenu au stade précédent, dans 8 ml de pyridine, ajoute 0,08 ml d'anhydre acétique et agite pendant 20 minutes à 0°C puis évapore la pyridine sous pression réduite. Les 340 mg de produit brut réactionnel obtenus sont directement engagés dans la réaction de réduction.

On refroidit sous atmosphère inerte à 0°C, 340 mg du produit brut dans 6 ml de tétrahydrofuranne, ajoute 96 mg de $LiAlH_4$ et agite 30 minutes à 0°C. On dilue le milieu dans un mélange acétate d'éthyle/eau et on extrait la phase aqueuse avec de l'acétate d'éthyle. La phase organique est séchée, évaporée sous pression réduite, et le résidu est chromatographié sur silice en éluant avec un mélange cyclohexane/acétate d'éthyle 80/20. On obtient ainsi 66 mg de produit attendu.
Rf = 0,31 cyclohexane/acétate d'éthyle 1/1
F=214°C
I.R. Nujol
absence de C=O
Absorption OH/NH possibles
Aromatiques 1610, 1590, 1516 $cm^{-1}$

**<u>EXEMPLE 36 :</u> 2,6-dichloro-4'-hydroxy-5'- (phénylméthyl)-(1,1'-biphényl)-4-méthanol**

<u>Stade A</u> : Déprotection + réarrangement

2,6-dichloro-4'-hydroxy-5'-(phénylméthyl)-(1,1'-biphényl)-4-carboxaldéhyde

On chauffe au reflux le mélange constitué de 522 mg de diphényle benzylé obtenu à l'exemple 24 stade D et 10 ml d'acide trifluoroacétique pendant 1 heure 30. Le milieu réactionnel est versé dans 100 ml d'eau, puis on extrait à l'acétate d'éthyle, sèche et évapore sous pression réduite. Le produit brut est purifié par chromatographie en phase inverse en éluant avec le mélange méthanol/eau 60/40 puis 80/20. On obtient 152 mg du produit attendu ainsi que 147 mg de l'analogue débenzylé.
Rf= 0,21 cyclohexane/acétate d'éthyle 80/20
RMN $CDCl_3$, 200MHz

| | |
|---|---|
| 4,05 s | C<u>H</u>$_2$Ph |
| 7,05 m 2H | $H_2$', $H_6$' |
| 6,95 d 1H | $H_3$' |
| 7,5 7,2 m 5H | benzyle |
| 7,8 s 2H | $H_3$, $H_5$ |
| 9,95 s 1H | C<u>H</u>O |

<u>Stade B</u> : réduction

2,6-diphényl-4'-hydroxy-5'-(phénylméthyl)-(1,1'-biphényl)-4-méthanol

On opère de manière équivalente à l'exemple 2 à partir de 140,4 mg de l'aldéhyde du stade précédent et on obtient 58 mg de produit attendu.
Rf = 0,27 cyclohexane/acétate d'éthyle 7/3
RMN $CDCl_3$, 200 MHz

| | | |
|---|---|---|
| 4,03 | s 2H | C<u>H</u>$_2$Ph |
| 7,03 | m 2H | $H_2$', $H_6$' |
| 6,86 | d 1H | $H_3$' |
| 7,16 | 7,34 m 5H | benzyle |
| 7,39 | s 2H | $H_3$, $H_5$ |
| 4,68 | sl 2H | C<u>H</u>$_2$OH |
| 1,86 | sl 1H | Ph-OH |
| 4,89 | sl 1H | $CH_2$O<u>H</u> |

En opérant de manière analogue aux exemples ci-dessus, à partir des composés appropriés, on a préparé les composés suivants :

**<u>EXEMPLE 37</u> : 2-bromo 6-[[4-[2-(diméthylamino) éthoxy] phényl] hydroxyméthyl] 4'-hydroxy (1,1'-biphényl) 4-méthanol.**

Rf = 0,19 ($CH_2Cl_2$/MeOH 8/2)

**<u>EXEMPLE 39</u> : 6'-bromo 4-[2-(diméthylamino) éthoxy] 4"-hydroxy (1,1':2',1"-terphényl) 4'-méthanol.**

Rf = 0,13 ($CH_2Cl_2$/MeOH 9/1)

**<u>EXEMPLE 40 :</u> 4-[2-(diméthylamino) éthoxy] 4"-hydroxy 6'-nitro (1,1':2',1"-torphényl) 4'-méthanol.**

Rf = 0,11 ($CH_2Cl_2$/MeOH 9/1)

**EXEMPLE 41 : 6'-chloro 4,4"-dihydroxy (1,1':2',1"-terphényl) 4'-méthanol.**

Rf = 0,36 ($CH_2Cl_2$/MeOH 9/1).

## COMPOSITION PHARMACEUTIQUE

On a préparé des comprimés répondant à la formule générale suivante :

- produit de l'exemple 7 50 mg
- Excipient (talc, amidon, stéarate de magnésium) qs pour un comprimé terminé à 120 mg

## ETUDE PHARMACOLOGIQUE DES PRODUITS DE L'INVENTION

1) Récepteur estrogène de l'utérus de rat (RER)

Des rats femelles de 280-300 g et castrées de 24 heures sont sacrifiées, les utérus sont prélevés puis homogénéisés à 0 C à l'aide d'un Potter téflon-verre dans une solution tamponnée TS (Tris 10mM, saccharose 0,25M, HCl pH 7,4) (1 g de tissu pour 10 ml de TS). L'homogénat est ensuite ultracentrifugé (209000 g x 30 mn) à 0 C. Des aliquotes du surnageant ainsi obtenu, sont incubés 24 heures à 0 C avec une concentration constante (2,5 $10^{-9}$M) d'estradiol tritié en présence de concentrations croissantes, soit d'estradiol froid (0-1000 x $10^{-9}$M), soit du produit froid à tester (0 - 25000 x $10^{-9}$M). La concentration d'estradiol tritié lié (B) est ensuite mesurée dans chaque incubat par la technique d'adsorption au charbon-dextran.

2) Récepteur estrogène humain (REH)

récepteur oestrogène humain recombinant est obtenu par surex-pression dans un système cellules d'insectes-Baculovirus, selon la méthodologie générale décrite par N.R. WEBB et al. (Journal of Methods in Cell and Molecular Biology, (1990) Vol.2 n°4, 173-188) et donc l'application est décrite à l'expression des récepteurs hormonaux humains, par exemple le récepteur glucocorticoïde humain (G. SRINIVASAN et al. Molecular Endocrinology (1990) vol 4 n°2 209-216).

On utilise le kit BaculoGold Transfection Kit (PharMingen, référence 21000K) pour générer le baculovirus recombinant contenant le fragment d'ADNc décrit dans le vecteur d'expression HEGO par L. TORA et al. (The EMBO Journal (1989) vol. 8 n° 7 1981-1986), comprenant la région codante pour le récepteur oestrogène humain de type sauvage avec une glycine en position 400.

Le virus recombinant ainsi obtenu est utilisé pour exprimer le récepteur estrogène dans les cellules d'insectes SF9 (ATCC CRL1711), selon la méthodologie connue citée précédemment.

$2*10^7$ cellules SF9 sont cultivées dans un flacon "Falcon" de 175 $cm^2$ dans le milieu TNM-FH "SIGMA" supplémenté avec 10 % de sérum de veau foetal (SVF) et avec 50 microgramme/ml de gentamycine. Après infection puis incubation à 27°C pendant 40 à 42 heures, les cellules sont lysées dans 1 ml de tampon de lyse (Tris 20 mM-HCl pH8, EDTA 0,5 mM, DTT 2 mM, Glycérol 20 %, KCl 400 mM) par un cycle de congélation-décongélation que l'on répète encore deux fois. Le surnageant, contenant le récepteur estrogène humain recombinant est conservé dans l'azote liquide par dose de 0,5 ml.

Le surnageant est incubé à 0°C pendant 24 heures (t long) ou 3 heures (t court) avec une concentration constante (T) d'oestradiol tritié en présence de concentrations croissantes soit d'estradiol froid (0 - 1000x$10^{-9}$M), soit du produit froid à tester (0 - 25000x$10^{-9}$M). La concentration d'estradiol tritié liée (B) est ensuite mesurée dans chaque incubat par la technique d'adsorption au charbon dextran.

Calcul de l'affinité relative de liaison (ARL) :

On trace les deux courbes suivantes : le pourcentage de l'hormone tritiée liée 100xB/B0 en fonction du logarithme de la concentration de l'hormone de référence froide ou en fonction du logarithme de la concentration du produit froid testé.

On détermine la droite d'équation

$$I_{50} = 100\ (B0/B0 + Bmin/B0)/2 = 100(1+Bmin/B0) = 50(1+Bmin/B0)$$

B0 = Concentration de l'hormone tritiée liée en l'absence de tout produit froid.
B = Concentration de l'hormone tritiée liée en présence d'une concentration X de produit froid.
Bmin = Concentration de l'hormone tritiée liée pour une incubation de cette hormone tritiée à la concentration (T) en présence d'un grand excès d'hormone froide de référence(1000x$10^{-9}$M) pour récepteur humain.
Les intersections de la droite $I_{50}$ et des courbes permettant d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation ARL=100(CH)/(CX).

Les résultats obtenus sont les suivants :

| Exemples | RER | REH estradiol=100 | |
|---|---|---|---|
| | | t courts | t long |
| (ex 2) | 12,5 | 425 | 74 |
| (ex 24) | 3 | 104 | 35 |
| (ex 30) | 2 | 24 | 5 |
| (ex 26) | - | - | 5,5 |
| (ex 1) | - | - | 5,5 |
| (ex 6) | - | - | 4,5 |
| (ex 25) | - | 12 | 5 |
| (ex 13) | 0,8 | 24 | 7,5 |
| (ex 28) | 2 | 33 | 8 |
| (ex 29) | 0,15 | - | 0,6 |
| (ex 4) | 4 | - | 22 |
| (ex 18) | 5 | - | 34 |
| (ex 36) | 8 | - | 0,07 |
| (ex 7) | 9, 5 | - | 50 |

Conclusion :

Certains de ces produits ont une affinité qui peut aller jusqu'à 425 % de l'estradiol ce qui est nouveau pour cette famille de molécule.

## Revendications

1. A titre de médicaments les composés de formule générale (1') :

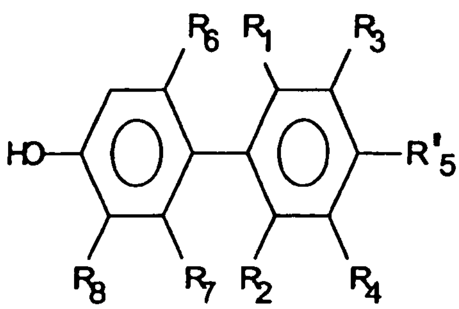

(I')

dans laquelle $R_1$ et $R_2$, identiques ou différents, représentent un atome d'hydrogène, un atome d'halogène, un radical hydroxyle, un radical trifluorométhyle, un radical nitro, un radical amino, un radical alkyloxy, alkylthio, alkylamino ou dialkylamino, dans lesquels alkyle renferme de 1 à 8 atomes de carbone, un groupement $-NR_AR_B$, dans lequel $R_A$ et $R_B$ forment avec l'atome d'azote auquel ils sont liés un hétérocycle saturé ou insaturé de 5 à 6 chaînons comportant éventuellement un autre hétéroatome choisi parmi N, O et S, un radical alkyle, alkényle ou alkynyle, linéaire ou ramifié, renfermant chacun au plus 8 atomes de carbone et éventuellement substitué, un radical aryle renfermant de 6 à 14 atomes de carbone et éventuellement substitué, un radical aralkyle renfermant de 7 à 15 atomes de carbone et éventuellement substitué, ou un radical CH(OH)-Y ou C(O)-Y dans lequel Y représente un radical alkyle, alkényle ou alkynyle renfermant de 1 à 8 atomes de carbone, substitué ou non substitué ou un groupement aryle renfermant de 6 à 14 atomes de carbone, substitué ou non substitué ou bien $R_1$ peut former avec $R_3$ un groupement -CH=CH-CH=CH-, $R_3$ et $R_4$, identiques ou différents, représentent un atome d'hydrogène, un atome d'halogène ou un radical alkyle renfermant de 1 à 8 atomes de carbone, ou $R_3$ peut former avec $R_1$ un groupement -CH=CH-CH=CH-, $R_6$ et $R_7$ identiques ou différents, représentent un atome d'hydrogène ou un atome d'halogène, $R_8$ représente un atome d'hydrogène ou un radical benzyle éventuellement substitué et $R'_5$ représente un radical $CH_2OH$, ainsi que les sels d'addition avec les acides ou les bases pharmaceutiquement acceptables.

**2.** A titre de médicaments, les composés de formule générale (I') telle que définie à la revendication 1, dans laquelle $R_6$, $R_7$ et $R_8$ sont des atomes d'hydrogène, ainsi que les sels d'addition avec les acides ou les bases pharmaceutiquement acceptables.

**3.** A titre de médicaments, les composés de formule générale (I') telle que définie à l'une quelconque des revendications 1 ou 2, dans laquelle $R_1$ et $R_2$ identiques ou différents sont des atomes d'halogènes et $R_3$, $R_4$, $R_6$, $R_7$ et $R_8$ sont des atomes d'hydrogène ainsi que les sels d'addition avec les acides et les bases pharmaceutiquement acceptables.

**4.** A titre de médicaments les composés de formule (I') telle que définie à la revendication 1, répondant à la formule générale (I") :

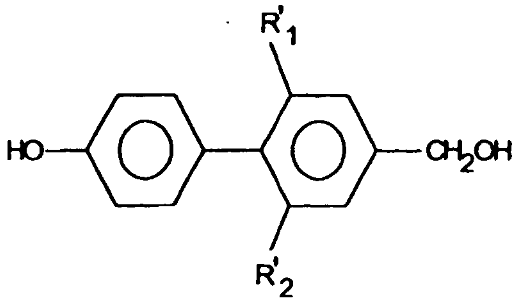

(I")

dans laquelle $R'_1$ représente un groupement aryle renfermant de 6 à 14 atomes de carbone et éventuellement substitué, $R'_2$ représente un atome d'halogène un radical nitro ou un radical amino, ainsi que les sels d'addition avec les acides et les bases pharmaceutiquement acceptables.

**5.** A titre de médicaments, les composés de formule générale (I") telle que définie à la revendication 4, dans laquelle $R'_1$ représente un radical phényle substitué par un groupement dialkylaminoalkyloxy ayant de 3 à 16 atomes de carbone,
ainsi que les sels d'addition avec les acides et les bases pharmaceutiquement acceptables.

**6.** A titre de médicaments, les composés de formule générale (I') telle que définie à la revendication 1, dont les noms suivent :

- 2,6-dibromo-4'-hydroxy-(1,1'-biphényl)-4-méthanol,
- 2,6-dichloro-4'-hydroxy-(1,1'-biphényl)-4-méthanol,
- 2,6-dinitro-4'-hydroxy-(1,1'-biphényl)-4-méthanol,
- 4,4"-dihydroxy-(1,1' : 2',1"-terphényl)-5'-méthanol,
- 2-bromo-4'-hydroxy-6-nitro-(1,1'-biphényl)-4-méthanol,
- 4'-hydroxy-2-trifluorométhyl-(1,1'-biphényl)-4-méthanol,
- 2-chloro-4'-hydroxy-6-(1-méthyléthyl)-(1,1'-biphényl)-4-méthanol,

- 2-chloro-4'-hydroxy-6-trifluorométhyl-(1,1'-biphényl)-4-méthanol,
- 2,6-dichloro-4'-hydroxy-5'-(phénylméthyl)-(1,1'-biphényl)-4-méthanol,
- 2-bromo 6-[[4-[2-(diméthylamino) éthoxy] phényl] hydroxyméthyl]4'-hydroxy(1,1'-biphényl)4-méthanol,
- 6'-bromo 4-[2-(diméthylamino) éthoxy] 4"-hydroxy (1,1':2',1"-terphényl)4'-méthanol,
- 4-[2-(diméthylamino) éthoxy] 4"-hydroxy 6'-nitro (1,1':2',1"-terphényl)4'-méthanol,
- 6'-chloro4,4"-dihydroxy(1,1':2',1"-terphényl) 4'-méthanol.

**7.** Compositions pharmaceutiques renfermant comme principe actif l'un au moins des médicaments tels que définis par l'une quelconque des revendications 1 à 6.

**8.** Les composés de formule générale (I') telle que définie à la revendication 1 ainsi que leurs sels d'addition avec les acides et les bases.

**9.** Les composés de formule générale (I') telle que définie aux revendications 2 à 6, ainsi que leurs sels d'addition avec les acides et les bases.

**10.** Les composés de formule générale (I") telle que définie à la revendication 4 ou 5, ainsi que leurs sels d'addition avec les acides et les bases.

**11.** Un procédé de préparation des produits de formule (I') telle que définie à la revendication 1 ou 8, **caractérisé en ce que** l'on soumet un produit de formule (II) :

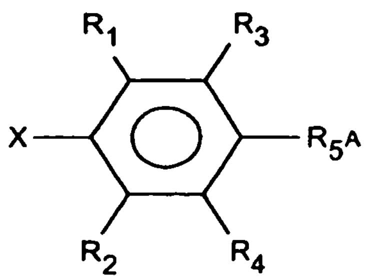

(II)

dans laquelle $R_1$, $R_2$, $R_3$ et $R_4$ sont tels que définis à la revendication 1 et $R_{5A}$ possède la valeur de $R'_5$ telle que définie à la revendication 1, ainsi que les valeurs hydrogène ou $-CO_2H$ estérifié ou non estérifié et X représente un atome d'hydrogène, d'halogène ou un groupement $OSO_2CF_3$, à l'action, en présence d'un catalyseur, d'un produit de formule (III) :

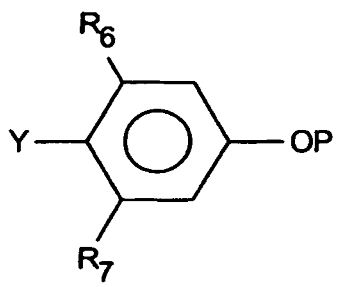

(III)

dans laquelle $R_6$ et $R_7$ sont tels que définis précédemment, Y représente un atome d'hydrogène, d'halogène, un groupement $B(OH)_2$ ou un groupement $Sn(R)_3$, dans lequel R représente un groupement alkyle renfermant de 1 à 8 atomes de carbone, et P représente un groupement protecteur , pour obtenir un produit de formule (IV):

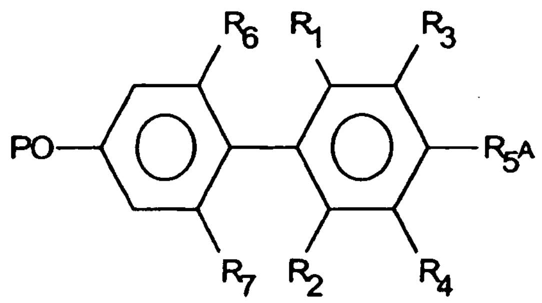

(IV)

dans laquelle P, $R_1$, $R_2$, $R_3$, $R_4$, $R_{5A}$, $R_6$ et $R_7$ ont la même signification que précédemment, produit de formule (IV) que si désiré ou si nécessaire l'on soumet dans un ordre approprié à l'une ou, le cas échéant, plusieurs des réactions suivantes afin d'obtenir le produit de formule (I') :

- déprotection du phénol,
- débenzylation puis réarrangement de façon à obtenir un produit de formule (I) avec $R_8$=benzyle,
- réduction totale ou partielle des groupements $NO_2$ que peuvent représenter $R_1$ ou $R_2$ en $NH_2$,
- substitution de $NH_2$ que peuvent représenter $R_1$, $R_2$, $R_3$ ou $R_4$ par Br ou par I,
- réaction de formylation lorsque $R_{5A}$ représente un atome d'hydrogène,
- réduction de la fonction $-CO_2H$ estérifiée que peut représenter $R_{5A}$,
- saponification,
- réduction du groupement -CHO que peut représenter $R_{5A}$ en un groupement $-CH_2OH$,
- oxydation du groupement -CHO que peut représenter $R_{5A}$ en un groupement $-CO_2H$,
- estérification du groupement $-CO_2H$ que peut représenter $R_{5A}$,

et salification par un acide ou une base.

**12.** Procédé de préparation des produits de formule (I")telle que définie à l'une quelconque des revendications 4, 5 et 10, **caractérisé en ce que** l'on soumet un composé de formule (V) :

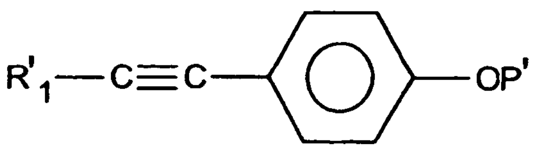

(V)

dans laquelle $R'_1$ est tel que défini à la revendication 4 et P' représente un radical alkyle renfermant de 1 à 4 atomes de carbone, à l'action d'un composé de formule (VI) :

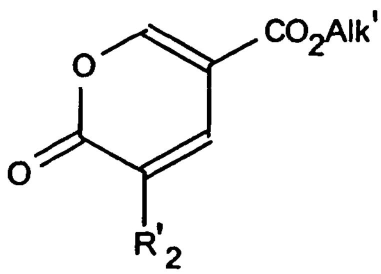

(VI)

dans laquelle Alk' représente un radical alkyle renfermant de 1 à 4 atomes de carbone et $R'_2$ est tel que défini à la revendication 4, afin d'obtenir un composé de formule (VII) correspondant à un produit de formule (IV) tel que défini à la revendication 11 :

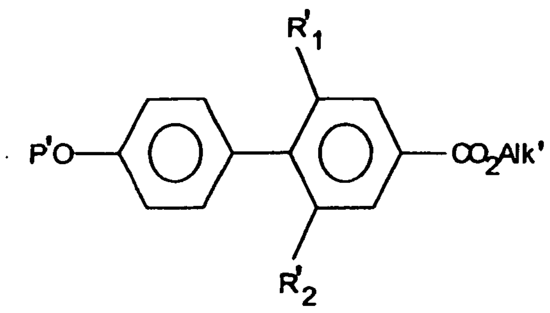

(VII)

dans lequel Alk', P', $R'_1$ et $R'_2$ sont tels que définis précédemment, puis à l'action d'un réactif de déprotection de la fonction hydroxy et de réduction de la fonction ester afin d'obtenir le produit de formule (I") et après, si désiré, à l'action d'une base ou d'un acide pour obtenir les sels correspondants.

**13.** A titre de produits industriels nouveaux, les produits de formule générale (IV) à l'exception du produit de formule (IV) dans laquelle $R_{5A}$ représente un radical [A]-$CO_2H$ estérifié ou non estérifié.

## Claims

**1.** As pharmaceutical products, the compounds according to formula :

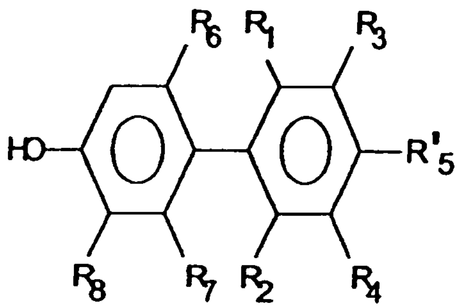

(I')

wherein

$R_1$ and $R_2$, identical or different,
are an hydrogen atom, an halogen atom, an hydroxyl, trifluoromethyl, nitro, amino, alkyloxy, alkylthio, alkylamino or dialkylamino radical, in which said alkyl comprise 1 to 8 carbon atoms,
a group $NR_AR_B$,
where $R_A$ and $R_B$ form with the nitrogen atom to which they are linked a saturated or unsaturated heterocyclic groups comprising 5 to 6 atoms, and optionally comprising another heteroatom chosen among N, O and S,
an alkyl, alkenyl or alkynyl linear or branched radical,
comprising each no more than 8 carbon atoms and being possibly branched,
an aryl radical
comprising 6 to 14 carbon atoms and optionally substituted,
an aralkyl radical comprising 7 to 15 carbon atoms and being possibly substituted
or
a radical CH(OH)-Y or C(O)-Y
where Y is an alkyl, alkenyl or alkynyl radical, substituted or not substituted, comprising 1 to 8 carbon atoms, or a group aryl, substituted or not substituted, comprising 6 to 14 carbon atoms,
or
$R_1$ together with $R_3$ can form a group -CH=CH-CH=CH-,
$R_3$ and $R_4$, identical or different,
are an hydrogen atom, an halogen atom or an alkyl radical comprising 1 to 8 carbon atoms

or $R_3$ together with $R_1$ can form a group -CH=CH-CH=CH-,
$R_6$ and $R_7$, identical or different, represent an hydrogen atom or an halogen atom,
$R_8$ represent an hydrogen atom or a benzyl radical, optionally substituted and
$R'_5$ is a $CH_2OH$ radical

as well as the addition salts with acids or bases pharmaceutically acceptable.

**2.** As pharmaceutical products, the compounds according to general formula (I') such as defined in claim 1, wherein $R_6$, $R_7$ and $R_8$ are hydrogen atoms, as well as the addition salts with acids or bases pharmaceutically acceptable.

**3.** As pharmaceutical products, the compounds according to general formula (I') such as defined in claim 1 or 2, wherein $R_1$ and $R_2$, identical or different are halogen atoms and $R_3$, $R_4$ $R_6$, $R_7$ and $R_8$ are hydrogen atoms, as well as the addition salts with acids or bases pharmaceutically acceptable.

**4.** As pharmaceutical products, the compounds according to general formula (I') such as defined in claim 1, according to general formula (I"):

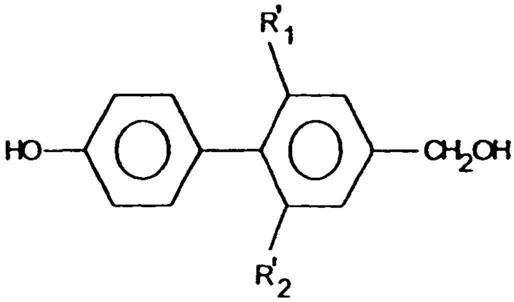

(I")

wherein $R'_1$ is a group aryl comprising 6 to 14 carbon atoms, being possibly substituted, $R'_2$ is a halogen atom, a nitro or an amino radical, as well as the addition salts with acids or bases pharmaceutically acceptable.

**5.** As pharmaceutical products, the compounds according to general formula (I") such as defined in claim 4, wherein $R'_1$ is a phenyl radical substituted by a dialkylaminoalkyloxy radical comprising 3 to 16 carbon atoms, as well as the addition salts with acids or bases pharmaceutically acceptable.

**6.** As pharmaceutical products, the compounds according to general formula (I') such as defined in claim 1, being

- 2,6-dibromo-4'-hydroxy-(1,1'-biphényl)-4-méthanol,
- 2,6-dichloro-4'-hydroxy-(I, 1'-biphényl)-4-méthanol,
- 2,6-dinitro-4'-hydroxy-(1,1'-biphényl)-4-méthanol,
- 4,4"-dihydroxy-(1,1':2',1 "-terphényl)-5'-méthanol,
- 2-bromo-4'-hydroxy-6-nitro-(1, 1'-biphényl)-4-méthanol,
- 4'-hydroxy-2-trifluoromethyl-(1,1'-biphenyl)-4-methanol,
- 2-chloro-4'-hydroxy-6-(1-methylethyl)-1,1'-biphenyl)-4-methanol,
- 2-chloro-4'-hydroxy-6-trifluoromethyl-(1,1'-biphenyl)-4-methanol,
- 2,6-dichloro-4'-hydroxy-5'-(phénylmethyl)-(1,1'-biphenyl)-4-méthanol,
- 2-bromo-6-[[4-[2-(diméthylamino)éthoxy]phényl]hydroxyméthyl]-4'-hydroxy-(1,1'-biphényl)-4-méthanol,
- 6'-bromo-4-[2-(diméthylamino)éthoxy]-4"-hydroxy-(1,1':2',1"-terphényl)-4'-methanol,
- 4-[2-(diméthylamino)éthoxy]-4"-hydroxy-6'-nitro-(1,1':2',1"-terphenyl)-4'-méthanol,
- 6'-chloro-4,4"-dihydroxy-(1,1':2',1"-terphényl)-4'-méthanol.

**7.** Pharmaceutical compositions comprising as active principle at least one of the pharmaceutical products, such as defined in any one of claims 1 to 6.

**8.** The compounds according to general formula (I') such as defined in claim 1, as well as the addition salts with acids or bases.

**9.** The compounds according to general formula (I') such as defined in claims 2 to 6, as well as the addition salts with acids or bases.

**10.** The compounds according to general formula (I") such as defined in claim 4 or 5, as well as the addition salts with acids or bases.

**11.** A process for the preparation of the compounds according to general formula (I') such as defined in claim 1 or 8, **characterized in that** a product according to formula (II)

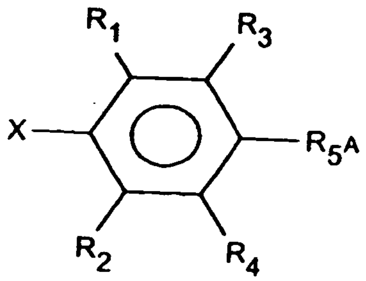

(II)

wherein

$R_1$, $R_2$, $R_3$ and $R_4$ are such as defined in claim 1 and
$R_{5A}$ presents the value than $R'_5$ such as defined in claim 1, or is an hydrogen atom or a group $-CO_2H$, esterified or not, and
X is an hydrogen atom, an halogen atom or a group $OSO_2\ CF_3$

is submitted to react, in the presence of a catalyst, with a compound according to formula (III):

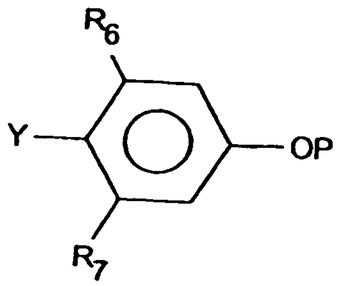

(III)

wherein

$R_6$ and $R_7$ are such as defined here above,
Y is an hydrogen atom, an halogen atom, a group $B(OH)_2$ or a group $Sn(R)_3$,
wherein R is a radical alkyl comprising 1 to 8 carbon atoms,
P is a protecting group,

to obtain a compound according to formula (IV):

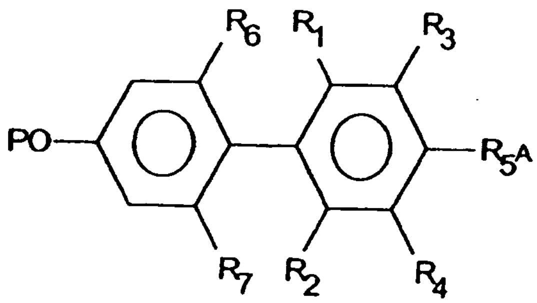

(IV)

wherein

P, $R_1$, $R_2$, $R_3$, $R_4$, $R_{5A}$, $R_6$ and $R_7$ are such as defined here above,

product of formula (IV) that if wished or necessary is submitted, in the adapted order, to one or possibly several of the following reactions, in order to obtain the compound according to formula (I'):

- deprotection of the phenol;
- debenzylation and rearrangement in order to obtain a compound according to formula (I) wherein $R_8$ is benzyl;
- total or partial reduction of the $NO_2$ groups corresponding to $R_1$ or $R_2$ into $NH_2$;
- substitution of $NH_2$ that $R_1$, $R_2$, $R_3$ or $R_4$ represent by Br or I;
- reaction of formylation when $R_{5A}$ is an hydrogen atom;
- reduction of the esterified $-CO_2H$ function that $R_{5A}$ can represent;
- saponification;
- reduction of the group -CHO that $R_{5A}$ can represent into a $CH_2OH$ group;
- oxydation of the group -CHO that $R_{5A}$ can represent into a $CO_2H$ group;
- esterification of the group $-CO_2H$ that $R_{5A}$ can represent;

and salification by an acid or a base.

**12.** Process for the preparation of the compounds according to general formula (I") such as defined in any one of claims 4, 5 or 10, **characterized in that** a compound according to formula (V)

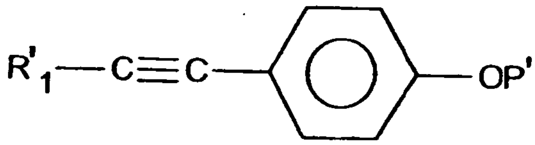

(V)

wherein

$R'_1$ is such as defined in claim 4, and
P' is an alkyl radical comprising 1 to 4 carbon atoms,
is submitted to a reaction with a compound according to formula (VI) :

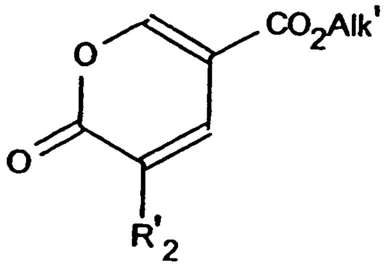

(VI)

wherein

Alk' is an alkyl radical comprising 1 to 4 carbon atoms, and
$R'_2$ is such as defined in claim 4,

in order to obtain a compound according to formula (VII) corresponding to a compound according to formula (IV), such as defined in claim 11,

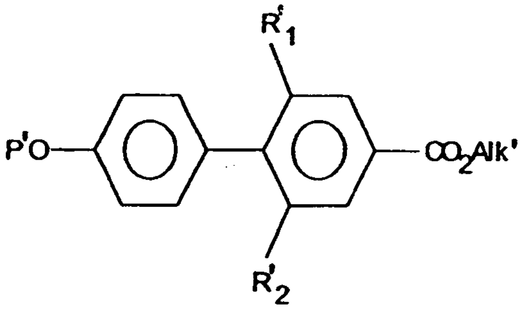

(VII)

wherein

Alk, P', $R'_1$ and $R'_2$ are such as defined here above,

and then to the action of an agent for the deprotection of the hydroxy function and the reduction of the ester function in order to obtain the product according to formula (I"), and then, possibly to the action of a base or of an acid to obtain the corresponding salts.

**13.** As new industrial products, the compounds according to general formula (IV) with the exception of the product of formula (IV) wherein $R_{5A}$ is an esterified or non esterified [A]-$CO_2H$ radical.

## Patentansprüche

**1.** Als Arzneimittel die Verbindungen der allgemeinen Formel (I'):

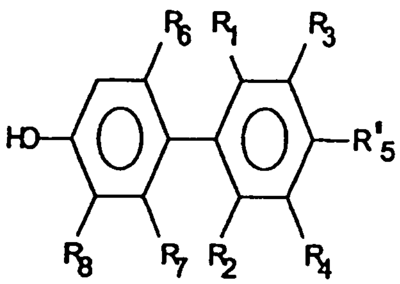

(I'),

worin $R_1$ und $R_2$ unabhängig voneinander ein Wasserstoffatom, ein Halogenatom, einen Hydroxylrest, einen Trifluormethylrest, einen Nitrorest, einen Aminorest, einen Alkyloxy-, Alkylthio-, Alkylamino- oder Dialkylaminorest, in denen Alkyl von 1 bis 8 Kohlenstoffatome enthält, eine Gruppe $-NR_AR_B$, in der $R_A$ und $R_B$ mit dem Stickstoffatom, an das sie gebunden sind, einen gesättigten oder ungesättigten Heterozyklus mit 5 bis 6 Ringgliedern bilden, der gegebenenfalls ein weiteres Heteroatom, ausgewählt aus N, O und S, enthält, einen Alkyl-, Alkenyl-, oder Alkinylrest, linear oder verzweigt, der jeweils höchstens 8 Kohlenstoffatome enthält und gegebenenfalls substituiert ist, einen Arylrest, der von 6 bis 14 Kohlenstoffatome enthält und gegebenenfalls substituiert ist, einen Aralkylrest, der von 7 bis 15 Kohlenstoffatome enthält und gegebenenfalls substituiert ist oder einen Rest CH(OH)-Y oder C(O)-Y, in dem Y einen Alkyl-, Alkenyl- oder Alkinylrest, der von 1 bis 8 Kohlenstoffatome enthält, substituiert oder nicht substituiert, darstellt, oder eine Arylgruppe, die von 6 bis 14 Kohlenstoffatome enthält, substituiert oder nicht substituiert, dar-

stellen oder $R_1$ mit $R_3$ eine Gruppe -CH=CH-CH=CH- bilden kann, $R_3$ und $R_4$ unabhängig voneinander ein Wasserstoffatom, ein Halogenatom oder einen Alkylrest, der von 1 bis 8 Kohlenstoffatome enthält, darstellen oder $R_3$ mit $R_1$ eine Gruppe -CH=CH-CH=CH- bilden kann, $R_6$ und $R_7$ unabhängig voneinander ein Wasserstoffatom oder ein Halogenatom darstellen, $R_8$ ein Wasserstoffatom oder einen Benzylrest, gegebenenfalls substituiert, darstellt und $R'_5$ einen Rest $CH_2OH$ darstellt, sowie die Additionssalze mit den pharmazeutisch verträglichen Säuren oder Basen.

**2.** Als Arzneimittel die Verbindungen der allgemeinen Formel (I'), wie sie in Anspruch 1 definiert ist, worin $R_6$, $R_7$ und $R_8$ Wasserstoffatome sind, sowie die Additionssalze mit den pharmazeutisch verträglichen Säuren oder Basen.

**3.** Als Arzneimittel die Verbindungen der allgemeinen Formel (I'), wie sie in einem beliebigen der Ansprüche 1 oder 2 definiert ist, worin $R_1$ und $R_2$ unabhängig voneinander Halogenatome sind und $R_3$, $R_4$, $R_6$, $R_7$ und $R_8$ Wasserstoffatome sind, sowie die Additionssalze mit den pharmazeutisch verträglichen Säuren und Basen.

**4.** Als Arzneimittel die Verbindungen der Formel (I'), wie sie in Anspruch 1 definiert ist, die der allgemeinen Formel (I") entsprechen:

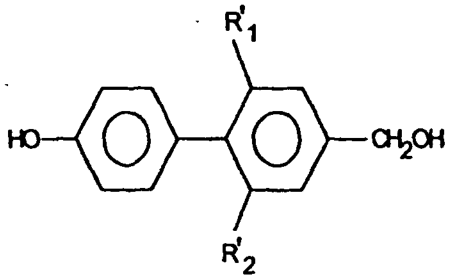

(I"),

worin $R'_1$ eine Arylgruppe darstellt, die von 6 bis 14 Kohlenstoffatome enthält und gegebenenfalls substituiert ist, $R'_2$ ein Halogenatom, einen Nitrorest oder einen Aminorest darstellt, sowie die Additionssalze mit den pharmazeutisch verträglichen Säuren und Basen.

**5.** Als Arzneimittel die Verbindungen der allgemeinen Formel (I"), wie sie in Anspruch 4 definiert ist, worin $R'_1$ einen Phenylrest darstellt, der mit einer Dialkylaminoalkyloxygruppe substituiert ist, die von 3 bis 16 Kohlenstoffatome aufweist, sowie die Additionssalze mit den pharmazeutisch verträglichen Säuren und Basen.

**6.** Als Arzneimittel die Verbindungen der allgemeinen Formel (I'), wie sie in Anspruch 1 definiert ist, deren Namen folgen:

- 2,6-Dibrom-4'-hydroxy-(1,1'-biphenyl)-4-methanol,
- 2,6-Dichlor-4'-hydroxy-(1,1'-biphenyl)-4-methanol,
- 2,6-Dinitro-4'-hydroxy-(1,1'-biphenyl)-4-methanol,
- 4,4"-Dihydroxy-(1,1' : 2',1"-terphenyl)-5'-methanol,
- 2-Brom-4'-hydroxy-6-nitro-(1,1'-biphenyl)-4'-methanol,
- 4'-Hydroxy-2-trifluormethyl-(1, 1'-biphenyl)-4-methanol,
- 2-Chlor-4'-hydroxy-6-(1-methylethyl)-(1,1'-biphenyl)-4-methanol,
- 2-Chlor-4'-hydroxy-6-trifluormethyl-(1,1'-biphenyl)-4-methanol,
- 2,6-Dichlor-4'-hydroxy-5'-(phenylmethyl)-(1,1'-biphenyl)-4-methanol,
- 2-Brom-6-[[4-[2-(dimethylamino)-ethoxy]-phenyl]-hydroxymethyl]-4'-hydroxy-(1,1'-biphenyl)-4-methanol,
- 6'-Brom-4-[2-(dimethylamino)-ethoxy]-4"-hydroxy-(1,1': 2',1 "-terphenyl)-4'-methanol,
- 4-[2-(dimethylamino)-ethoxy]- 4"-hydroxy-6'-nitro-(1,1': 2',1 "-terphenyl)-4'-methanol,
- 6'-Chlor-4,4"-dihydroxy-(1,1': 2',1"-terphenyl)-4'-methanol.

**7.** Pharmazeutische Zusammensetzungen, die als Wirkstoff wenigstens eines der Arzneimittel enthalten, wie sie in einem beliebigen der Ansprüche 1 bis 6 definiert sind.

**8.** Die Verbindungen der allgemeinen Formel (I'), wie sie in Anspruch 1 definiert ist, sowie deren Additionssalze mit den Säuren und den Basen.

**9.** Die Verbindungen allgemeinen Formel (I'), wie sie in den Ansprüchen 2 bis 6 definiert ist, sowie deren Additionssalze mit den Säuren und den Basen.

**10.** Die Verbindungen der allgemeinen Formel (I"), wie sie in Anspruch 4 oder 5 definiert ist, sowie deren Additionssalze mit den Säuren und den Basen.

**11.** Verfahren zur Herstellung von Produkten der Formel (I'), wie sie in Anspruch 1 oder 8 definiert ist, **dadurch gekennzeichnet, dass** man ein Produkt der Formel (II):

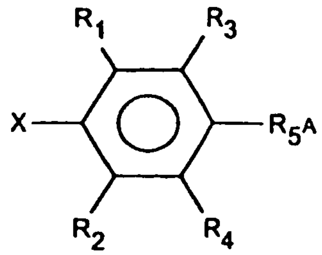

(II),

worin $R_1$, $R_2$, $R_3$ und $R_4$ wie in Anspruch 1 definiert sind und $R_{5A}$ die Bedeutung von $R'_5$, wie sie in Anspruch 1 definiert ist, sowie die Bedeutungen Wasserstoff oder $-CO_2H$, verestert oder nicht verestert, besitzt und X ein Wasserstoff- oder Halogenatom oder eine Gruppe $OSO_2CF_3$ darstellt, in der Gegenwart eines Katalysators der Wirkung eines Produkts der Formel (III):

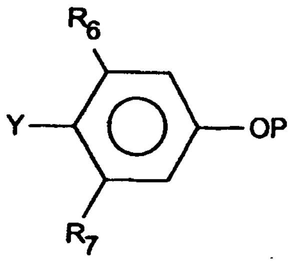

(III)

unterzieht, worin $R_6$ und $R_7$ so wie zuvor definiert sind, Y ein Wasserstoff-, ein Halogenatom, eine Gruppe $B(OH)_2$ oder eine Gruppe $Sn(R)_3$ darstellt, worin R eine Alkylgruppe darstellt, die von 1 bis 8 Kohlenstoffatome enthält, und P eine Schutzgruppe darstellt, um ein Produkt der Formel (IV):

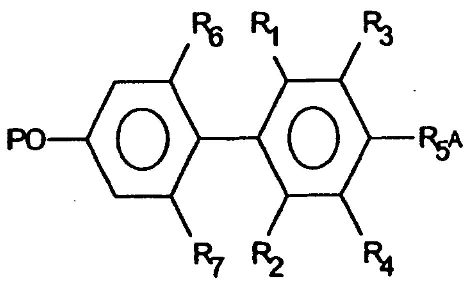

(IV)

zu erhalten, worin P, $R_1$, $R_2$, $R_3$, $R_4$, $R_{5A}$, $R_6$ und $R_7$ die gleiche Bedeutung wie zuvor besitzen, wobei das Produkt

der Formel (IV) erwünscht ist oder man es falls erforderlich in einer geeigneten Reihenfolge einer oder gegebenenfalls mehreren der folgenden Reaktionen unterzieht, um das Produkt der Formel (I') zu erhalten:

- Entfernen der Schutzgruppe von dem Phenol,
- Debenzylierung mit anschließender Umlagerung, derart, um ein Produkt der Formel (I) mit $R_8$ = Benzyl zu erhalten,
- vollständige oder teilweise Reduktion der Gruppen $NO_2$, die $R_1$ oder $R_2$ darstellen können, zu $NH_2$,
- Substitution von $NH_2$, die $R_1$, $R_2$, $R_3$ oder $R_4$ darstellen können, durch Br oder durch I,
- Formylierungsreaktion, wenn $R_{5A}$ ein Wasserstoffatom darstellt,
- Reduktion der veresterten Funktion $-CO_2H$ die $R_{5A}$ darstellen kann,
- Verseifung,
- Reduktion der Gruppe -CHO, die $R_{5A}$ darstellen kann, zu einer Gruppe
- $CH_2OH$,
- Oxidation der Gruppe -CHO, die $R_{5A}$ darstellen kann, zu einer Gruppe $-CO_2H$,
- Veresterung der Gruppe $-CO_2H$, die $R_{5A}$ darstellen kann,

und Salzbildung durch eine Säure oder eine Base.

**12.** Verfahren zur Herstellung von Produkten der Formel (I"), wie sie in einem beliebigen der Ansprüche 4, 5 und 10 definiert ist, **dadurch gekennzeichnet, dass** man eine Verbindung der Formel (V):

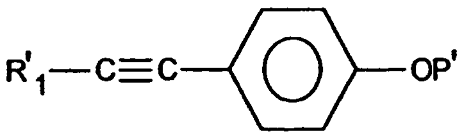

(V),

worin $R'_1$ wie in Anspruch 4 definiert ist und P' einen Alkylrest darstellt, der von 1 bis 4 Kohlenstoffatome enthält, der Wirkung einer Verbindung der Formel (VI):

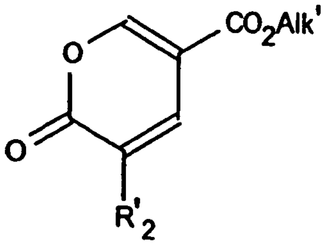

(VI)

unterzieht, worin Alk' einen Alkylrest darstellt, der von 1 bis 4 Kohlenstoffatome enthält, und $R'_2$ wie in Anspruch 4 definiert ist, um eine Verbindung der Formel (VII) zu erhalten, die einem Produkt der Formel (IV) entspricht, wie es in Anspruch 11 definiert ist:

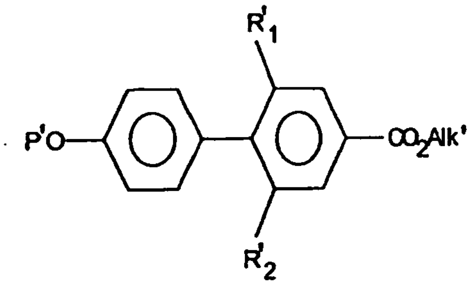

(VII),

worin Alk', P', $R'_1$ und $R'_2$ wie zuvor definiert sind, und dann der Wirkung eines Reagenz für das Entfernen der Schutzgruppe der Hydroxyfunktion und die Reduktion der Esterfunktion unterzieht, um das Produkt der Formel (I") zu erhalten, und danach, falls erwünscht, der Wirkung einer Base oder einer Säure unterzieht, um die entsprechenden Salze zu erhalten.

**13.** Als neue industrielle Produkte die Produkte der allgemeinen Formel (IV) mit der Ausnahme des Produkts der Formel (IV), worin $R_{5A}$ einen Rest [A]-$CO_2H$, verestert oder nicht verestert, darstellt.